# EUROPEAN PATENT SPECIFICATION

(11) **EP 4 138 730 B1**
(45) Date of publication and mention of the grant of the patent: **15.05.2024**
(21) Application number: 21736897.6
(22) Date of filing: 10.06.2021
(51) Int. Cl.: A61F 2/24, A61F 2/95, A61F 2/966, A61M 25/01

(54) **RELEASE MECHANISM FOR A DELIVERY APPARATUS FOR AN IMPLANTABLE MEDICAL DEVICE**
FREIGABEMECHANISMUS FÜR EINE FREISETZUNGSVORRICHTUNG FÜR EINE IMPLANTIERBARE MEDIZINISCHE VORRICHTUNG
MÉCANISME DE LIBÉRATION POUR UN APPAREIL D'ADMINISTRATION POUR UN DISPOSITIF MÉDICAL IMPLANTABLE

(30) Priority: 10.06.2020 US 202063037501 P
(43) Date of publication of application: 01.03.2023
(73) Proprietor: Edwards Lifesciences Corporation, Irvine, CA 92614-5688 (US)
(72) Inventor: GAFFNEY, Leah Paige, Orange, CA 92866 (US); VALENCIA, Salomon Xavier, Irvine, CA 92614 (US); RAFI, Hamid, Irvine, CA 92614 (US); MANZELLA, Salvatore, Jr., Irvine, CA 92614 (US)
(74) Representative: Eisenführ Speiser
(86) International application number: PCT/US2021/036717
(87) International publication number: WO 2021/252707

(56) References cited:
- US-A1- 2015 282 965
- US-A1- 2019 008 639

## Description

### CROSS REFERENCE TO RELATED APPLICATION

This application claims the benefit of U.S. Provisional Patent Application No. 63/037,501, filed June 10, 2020.

### FIELD

The present disclosure relates to embodiments of a release mechanism for a handle of a delivery apparatus for an implantable medical device, such as a prosthetic heart valve.

### BACKGROUND

Delivery apparatuses, such as endovascular delivery apparatuses, are used in various procedures to deliver prosthetic medical devices to locations inside the body that are not readily accessible by surgery or where access without surgery is desirable. Access to a target location inside the body is achieved by a medical specialist inserting and guiding the delivery apparatus through a pathway or lumen in the body, including, but not limited to, a blood vessel, an esophagus, a trachea, any portion of the gastrointestinal tract, a lymphatic vessel, to name a few. The prosthetic medical device may include expandable valves or instruments (e.g., stents). In one specific example, an expandable prosthetic heart valve can be mounted in a radially compressed (or crimped) state on a distal end of the delivery apparatus and then deployed from a capsule of the delivery apparatus at the implantation site so that the prosthetic valve can self-expand to its functional size.

In some embodiments, the delivery apparatus can include an articulating portion with one or more steering mechanism that allow a distal end portion of the delivery apparatus to articulate (e.g., curve or flex) when being guided through a patient's vasculature. For example, at least a distal end portion of the delivery apparatus may be required to articulate over the aortic arch in order to deliver a prosthetic aortic valve arranged on the distal end of the delivery apparatus to its target implantation site. The delivery apparatus can include multiple shafts with concentric lumens that may shorten or lengthen with respect to each other as the distal end portion of the delivery apparatus articulates/flexes.

In some embodiments, after flexing the distal end portion of the delivery apparatus to reach the target implantation site, and while the distal end portion remains flexed, the valve can be released from the delivery apparatus by rotating a knob of a release mechanism of the delivery apparatus. This causes linear movement (proximally, along the axial direction) of an inner shaft coupled to release members that are releasably coupled with the valve. However, this linear translation of concentric lumens when flexed can cause shortening of the inner shaft, thereby creating tension in the distal end portion of the delivery apparatus upon unflexing. If the release mechanism is not unlocked (via a locking mechanism) to relieve this tension during unflexing, the distal end portion can be held in tension, thereby preventing the delivery apparatus from being removed from the implantation site.

Such locking mechanisms may increase the complexity of the implantation procedure and create tension issues that may increase the difficulty in removing the delivery apparatus from the implantation site, after implanting the valve.

Accordingly, a need exists for improved delivery apparatuses that can relieve tension created during releasing the valve from the delivery apparatus, prior to unflexing the catheter.

US 2019/0008639 describes devices, systems and methods to provide improved steerability for delivering a prosthesis to a body location, for example, for delivering a replacement mitral valve to a native mitral valve location. Disclosed are a number of features that can improve steerability or release of a prosthesis into the body location.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention.

Also disclosed herein are examples of an improved delivery apparatus for an implantable medical device (e.g., a prosthetic heart valve), as well as related methods for use of such an apparatus in implanting an implantable medical device in a patient's body. In some examples, the delivery apparatus can include a handle portion which a user (e.g., physician, medical technician, or the like) can hold and use to operate the delivery apparatus. In some examples, the handle portion can include a release mechanism configured to adjust a linear position of a component of the delivery apparatus, the handle portion including a rotatable knob and a drive screw arranged within the knob. The knob and drive screw can be mated such that rotation of the knob results in linear translation of the drive screw and the component of the delivery apparatus. In some examples, the knob and drive screw can be configured to automatically relieve tension in the distal end portion of the delivery apparatus, during an implantation procedure.

The presently claimed invention provides a delivery apparatus for an expandable, implantable medical device including: a handle portion including a release mechanism configured to adjust a linear position, relative to a central longitudinal axis of the delivery apparatus, of a component of the delivery apparatus, the release mechanism comprising: a threaded drive screw including a helical threaded portion having a lead of at least 2.5cm (one inch), where the helical threaded portion includes one or more grooves extending around the drive screw, the drive screw coupled to the component; and a rotatable knob surrounding and coaxial with the drive screw, the knob including one or more teeth arranged at a proximal end of the knob, each tooth of the one or more teeth configured to interface with a corresponding groove of the one or more grooves of the drive screw, wherein each tooth of the one or more teeth extends from the proximal end, toward a distal end of the knob, only a portion of a total distance between the proximal end and the distal end, wherein the portion is less than ¼ the total distance.

Also disclosed herein is a method not covered by the present invention, for implanting an implantable medical device with a delivery apparatus including: advancing a distal end portion of a delivery apparatus to a target implantation site using a handle portion of the delivery apparatus, the implantable medical device arranged in a radially compressed configuration on the distal end portion; and after reaching the target implantation site, uncovering the radially compressed implantable medical device and releasing the implantable medical device from the delivery apparatus, the releasing including: from a starting position, rotating a knob of a release mechanism of the handle portion of the delivery apparatus and moving one or more teeth of the knob along one or more corresponding grooves of a drive screw of the release mechanism to linearly translate the drive screw in a proximal direction, along an axis that is parallel to a central longitudinal axis of the delivery apparatus, until the drive screw reaches a released position; while and as a result of the drive screw translating in the proximal direction, linearly translating an inner shaft fixedly coupled with the drive screw and one or more release members fixedly coupled to a distal end portion of the inner shaft to release the implantable medical device from the delivery apparatus; and actuating a steering mechanism of the delivery apparatus to unflex a distal end portion of the delivery apparatus and passively retracting the drive screw in a distal direction, partially into the knob to automatically release tension during the unflexing, the distal direction opposite to the proximal direction.

Also disclosed herein is a method not covered by the present invention, for operating a release mechanism of a handle portion of a delivery apparatus configured to deliver an implantable medical device to a target implantation site, including: from a starting, locked position of the release mechanism, moving one or more teeth of a knob of the release mechanism along one or more corresponding grooves of a drive screw of the release mechanism to linearly translate the drive screw in a proximal direction, along an axis that is parallel to a central longitudinal axis of the delivery apparatus, until the drive screw reaches a released position, in response to rotation of the knob, wherein in the starting, locked position a main body of the drive screw including the one or more grooves is arranged within an interior of the knob and in the released position, a majority of the main body extends outside of the knob; while the drive screw is translating in the proximal direction, linearly translating an inner shaft fixedly coupled with the drive screw and one or more release members fixedly coupled to a distal end portion of the inner shaft to release the implantable medical device mounted on the distal end portion of the delivery apparatus from the delivery apparatus; and unflexing the distal end portion of the delivery apparatus in response to actuation of a steering mechanism of the delivery apparatus and, during the unflexing, passively retracting the drive screw in a distal direction, partially into the knob to automatically release tension in the distal end portion of the delivery apparatus and enable the unflexing, the distal direction opposite the proximal direction.

One aspect of the disclosure provides a delivery apparatus for an expandable, implantable medical device including: an inner shaft; one or more release members, each including a proximal end coupled to an outer surface of a distal end portion of the inner shaft and a distal end configured to be releasably coupled to the implantable medical device arranged around the distal end portion of the inner shaft, distal to where the proximal end couples to the inner shaft; and a handle portion, including: a steering mechanism configured to adjust a curvature of and flex one or more shafts of the delivery apparatus, including the inner shaft, at a distal end portion of the delivery apparatus; and a release mechanism configured to adjust a linear position of the inner shaft and the one or more release members, along a central longitudinal axis of the delivery apparatus, relative to an outer housing of the handle portion, the release mechanism comprising: a threaded drive screw coupled to a proximal end of the inner shaft and including a helical threaded portion arranged in a main body of the drive screw, where one or more grooves forming the helical threaded portion extend from a proximal end to a distal end of the main body of the drive screw; and a rotatable release knob coupled to the housing of the handle portion and surrounding and coaxial with the drive screw, the release knob including one or more teeth arranged at a proximal end of the knob and configured to interface with the one or more grooves of the drive screw The drive screw is adapted to move linearly, along the central longitudinal axis, relative to the release knob, in response to rotation of the release knob and sliding of the one or more teeth along the one or more grooves, and actuating the steering mechanism to unflex the one or more shafts of the delivery apparatus allows the drive screw to move distally, along the central longitudinal axis, to release tension created in the distal end portion of the delivery apparatus.

The foregoing and other objects, features, and advantages of the invention will become more apparent from the following detailed description, which proceeds with reference to the accompanying figures.

### BRIEF DESCRIPTION OF THE DRAWINGS

FIG. 1 is a side elevation view of an exemplary embodiment of an implantable prosthetic heart valve that can be implanted using any of the delivery apparatuses disclosed herein.
FIG. 2 is a side elevation view of an exemplary embodiment of a delivery apparatus for delivering the prosthetic heart valve of FIG. 1.
FIG. 3 is a side cross-sectional view of the distal end portion of the delivery apparatus of FIG. 2 showing the prosthetic valve housed in a compressed state within a delivery capsule.
FIG. 4 is a side elevation view of the distal end portion of the delivery apparatus of FIG. 2 shown with the capsule of the delivery apparatus advanced over a portion of the prosthetic heart valve frame.
FIG. 5 is a side elevation view of the handle portion of the delivery apparatus of FIG. 2.
FIG. 6 is a side view of the handle portion of FIG. 5 with half of the housing of the handle portion removed to show internal components of the handle portion.
FIG. 7 is a side cross-sectional view of the handle portion of the delivery apparatus of FIG. 2, showing a portion of the internal components of the handle portion.
FIG. 8 is a side cross-sectional view of a portion of a handle portion of a delivery apparatus, according to an embodiment, which includes a locking mechanism for a valve release mechanism of the handle portion.
FIG. 9 is a side view of a distal end portion of a delivery apparatus, such as the delivery apparatus of FIG. 2, in a starting configuration prior to releasing a prosthetic heart valve from the delivery apparatus.
FIG. 10 is a side view of the distal end portion of the delivery apparatus of FIG. 9, in a released configuration, after releasing the prosthetic heart valve from the delivery apparatus.
FIG. 11 is an exemplary schematic of a distal end portion of a delivery apparatus articulating around a simulated aortic arch, en route to a target implantation site for a prosthetic medical device arranged on the distal end portion.
FIG. 12 is a side elevation view of an embodiment of a handle portion of a delivery apparatus, the handle portion including a release mechanism configured to automatically relieve tension in a distal end portion of the delivery apparatus.
FIG. 13 is a side cross-sectional view of the handle portion of FIG. 12, with the release mechanism in a starting, locked configuration.
FIG. 14 is a side elevation view of the handle portion of FIG. 12, with a steering mechanism knob and a cap of the release mechanism removed.
FIG. 15 is a side cross-sectional view of the handle portion of FIG. 14, showing the release mechanism in the starting, locked configuration.
FIG. 16 is a side elevation view of the handle portion of FIG. 12, with the steering mechanism knob removed and showing the release mechanism in a released configuration.
FIG. 17 is a side cross-sectional view of the handle portion of FIG. 16, showing the release mechanism in the released configuration.
FIG. 18 is another side cross-sectional view of the handle portion of FIG. 16, showing the release mechanism in the released configuration.
FIG. 19 is a perspective cross-sectional view of a portion of the release mechanism of the handle portion of FIG. 12, showing an end-of-travel feature of the release mechanism.
FIG. 20 is a side elevation view of the handle portion of FIG. 16, showing the release mechanism in a partially retracted configuration, while automatically relieving tension during an implantation procedure with the delivery apparatus.
FIG. 21 is a perspective view, from a distal end, of a rotatable knob of the release mechanism of FIG. 12
FIG. 22 is a side cross-sectional view of the knob of FIG. 21.
FIG. 23 is a proximal end view of the knob of FIG. 21.
FIG. 24 is a perspective, top view of the knob of FIG. 21.
FIG. 25 is a perspective view, from a proximal end, of the knob of FIG. 21.
FIG. 26 is a perspective view of a drive screw of the release mechanism of FIG. 12.
FIG. 27 is a top view of the drive screw of FIG. 26.
FIG. 28 is a proximal end view of the drive screw of FIG. 26.
FIG. 29 is a side cross-sectional view of the drive screw, taken along section A-A of FIG. 28.
FIG. 30 is a side elevation view of the drive screw of FIG. 26.
FIG. 31 is a first, end cross-sectional view of the drive screw, taken along section B-B of FIG. 30.
FIG. 32 is a second, end cross-sectional view of the drive screw, taken along section C-C of FIG. 30.
FIG. 33 is a distal end view of the drive screw of FIG. 26.
FIG. 34 is a flow chart of a method, not covered by the present invention, for operating a handle portion of a delivery apparatus, such as the handle portion of FIG. 12, to deliver a prosthetic medical device to a target implantation site.

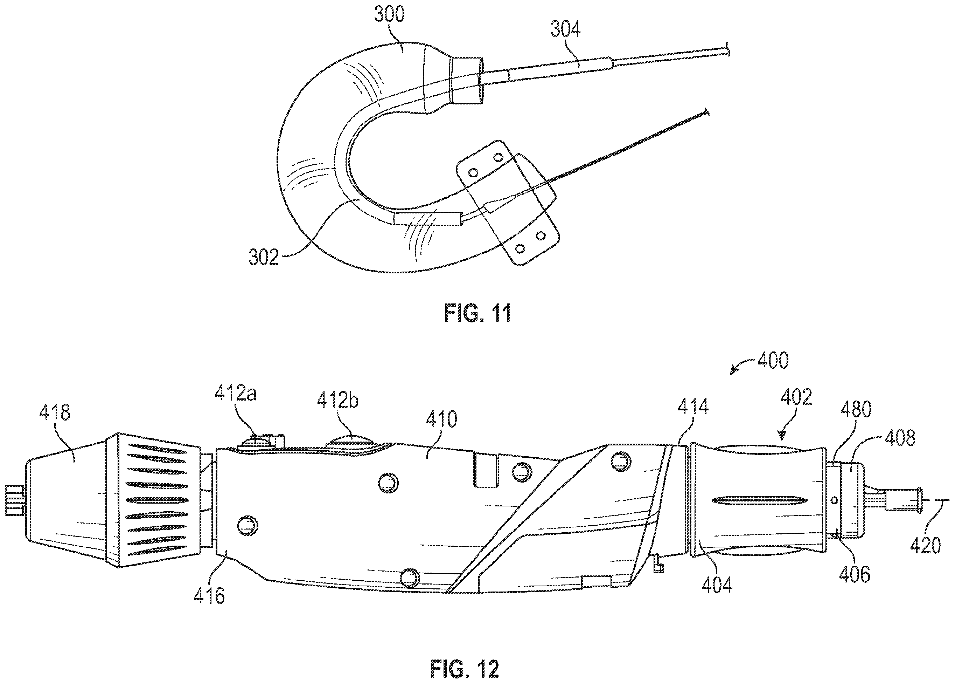

The invention relates to a delivery apparatus as illustrated on fig. 12-33.

### DETAILED DESCRIPTION

### General Considerations

For purposes of this description, certain aspects, advantages, and novel features of the embodiments of this disclosure are described herein. The described methods, systems, and apparatus should not be construed as limiting in any way. Instead, the present disclosure is directed toward all novel and non-obvious features and aspects of the various disclosed embodiments, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present, or problems be solved.

Features, integers, characteristics, compounds, chemical moieties, or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods, systems, and apparatus can be used in conjunction with other systems, methods, and apparatus.

As used herein, the terms "a," "an," and "at least one" encompass one or more of the specified element. That is, if two of a particular element are present, one of these elements is also present and thus "an" element is present. The terms "a plurality of' and "plural" mean two or more of the specified element.

As used herein, the term "and/or" used between the last two of a list of elements means any one or more of the listed elements. For example, the phrase "A, B, and/or C" means "A," "B," "C," "A and B," "A and C," "B and C," or "A, B, and C."

As used herein, the term "coupled" generally means physically coupled or linked and does not exclude the presence of intermediate elements between the coupled items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

As used herein, with reference to the prosthetic heart valve and the delivery apparatus, "proximal" refers to a position, direction, or portion of a component that is closer to the user and/or a handle of the delivery apparatus that is outside the patient, while "distal" refers to a position, direction, or portion of a component that is further away from the user and/or the handle of the delivery apparatus and closer to the implantation site. The terms "longitudinal" and "axial" refer to an axis extending in the proximal and distal directions, unless otherwise expressly defined. Further, the term "radial" refers to a direction that is arranged perpendicular to the axis and points along a radius from a center of an object (where the axis is positioned at the center, such as the longitudinal axis of the prosthetic valve).

### Examples of the Disclosed Technology

Described herein are examples of a delivery apparatus that can be used to deliver an implantable, expandable medical device, such as a prosthetic heart valve, to a target implantation site in a patient. The delivery apparatus can include a handle portion and one or more concentric shafts extending distally away from the handle portion. The implantable medical device can be mounted in a radially compressed configuration on a distal end portion of the delivery apparatus. For example, a delivery capsule may be coupled to an outer shaft of the delivery apparatus, at the distal end portion, and cover and retain the implantable medical device in its radially compressed state thereon. The handle portion can include a housing and one or more buttons and/or knobs that are actuatable by a user and configured to adjust operation of the delivery apparatus, during an implantation procedure. In some embodiments, the handle portion can include a steering knob configured to adjust an amount of curvature of one or more shafts of the delivery apparatus, at the distal end portion of the delivery apparatus, thereby allowing the implantable medical device to be delivered through curving lumens of a patient.

In some embodiments, the handle portion can include a valve release mechanism that is configured to adjust a linear position (along a central longitudinal axis of the delivery apparatus) of one or more components of the delivery apparatus and automatically relieve tension created at the distal end portion of the delivery apparatus during curving the distal end portion and linearly adjusting the position of one or more components at the distal end portion. For example, in some embodiments, the steering mechanism is configured to adjust a linear position of one or more release members coupled with the implantable medical device, in order to release the implantable medical device from the delivery apparatus. In some embodiments, the release mechanism includes a rotatable knob coupled to the housing of the handle portion and a threaded drive screw arranged within an interior of the knob. Rotation of the knob can cause the drive screw to translate in the axial direction via a mating connection between one or more grooves of the drive screw and one or more teeth of the knob. The drive screw may be coupled to an inner shaft coupled to the one or more release member. Thus, linear movement of the drive screw can result in concurrent linear movement of the inner shaft and one or more release members. A pitch and lead of the threads of the drive screw (formed by the one or more grooves) can be relatively long while a length of the teeth of the knob are relatively short. As a result, after uncoupling the release members from the implantable medical device, via manually rotating the release mechanism knob, and while unflexing the distal end portion of the delivery apparatus (e.g., via the steering mechanism), the drive screw may passively translate in the distal, axial direction and retract back into the knob, thereby relieving tension at the distal end portion of the delivery apparatus and facilitating the unflexing. As a result, the delivery apparatus may be more easily removed from the implantation site and the overall implantation procedure with the delivery apparatus may be simplified.

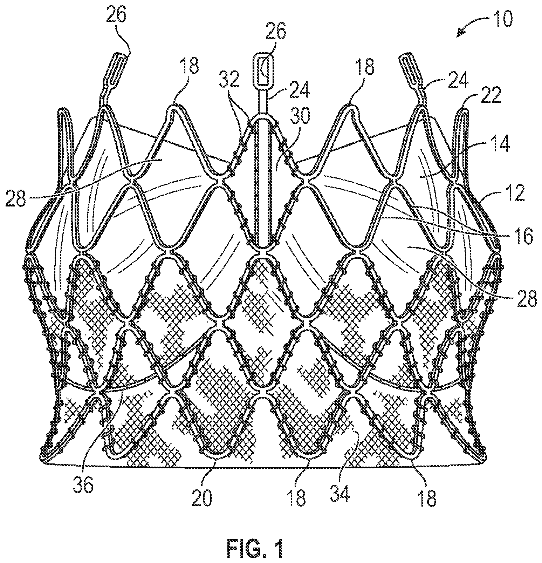

In some embodiments, the delivery apparatus is configured to deliver and implant a prosthetic heart valve, such as the example prosthetic heart valve of FIG. 1, at a selected implantation site within a patient (e.g., within the native aortic valve, mitral valve, tricuspid valve or pulmonary valve). In addition to prosthetic heart valves, disclosed delivery apparatuses can be adapted to deliver and implant other types of prosthetic valves (e.g., venous valves) and various other types of prosthetic devices, such as stents, grafts, docking devices for prosthetic heart valves, heart valve repair devices (e.g., leaflet clips), embolic coils, and the like; to position imaging devices and/or components thereof, including ultrasound transducers; and to position energy sources, for example, devices for performing lithotripsy, RF sources, ultrasound emitters, electromagnetic sources, laser sources, thermal sources, and the like.

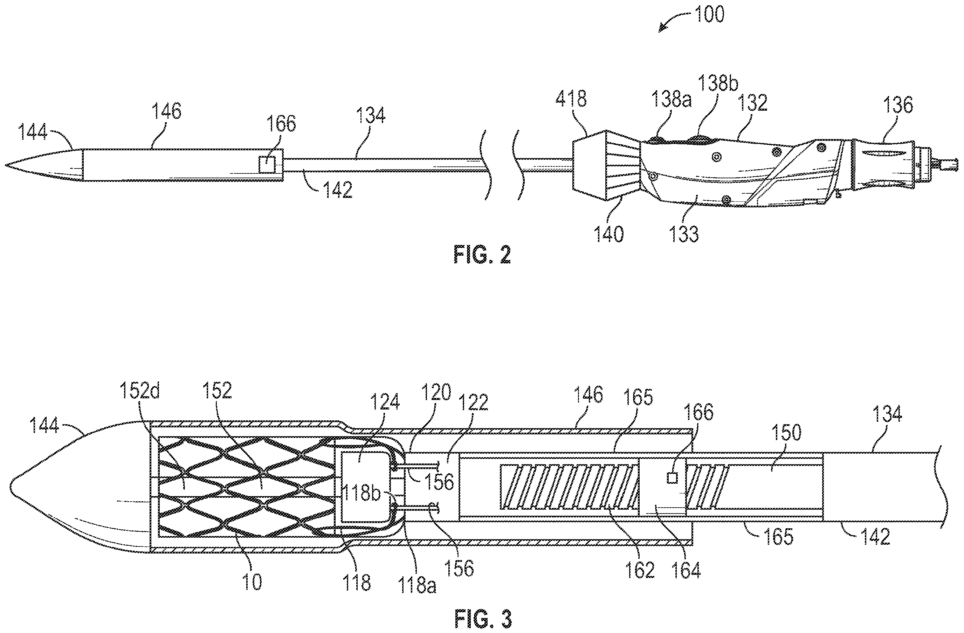

FIG. 1 shows a prosthetic heart valve 10, according to one embodiment, that can be implanted with a delivery apparatus, such as delivery apparatus 100 of FIG. 2. In some embodiments, the prosthetic heart valve is a self-expanding valve that is delivered in a radially compressed state to a deployment site via the delivery apparatus. When advanced from a delivery capsule at the distal end of the delivery apparatus (e.g., the delivery apparatus of FIG. 2), the prosthetic valve can radially self-expand to its functional size.

The prosthetic heart valve 10 comprises a stent, or frame 12 and a valvular structure 14 (e.g., leaflets or a flap valve) supported by the frame 12. The frame 12 can have a plurality of interconnected struts 16 arranged in a lattice-like pattern and forming a plurality of apices 18 at the inflow and outflow ends 20, 22, respectively, of the frame 12

The frame 12 can include a plurality of angularly-spaced posts 24 extending from respective apices 18 at the outflow end of the frame 12. The frame 12 in the illustrated embodiment includes three such posts 24, although a greater or fewer number of posts can be used. In one implementation, the frame 12 can have posts 24 extending from all the apices 18 at the outflow end 22 of the frame 12. Each post 24 can have an eyelet or aperture 26, which can be used to form a releasable connection with the delivery apparatus (e.g., delivery apparatus 100), such as via the use of one or more cords or tethers 118 (see FIG. 3, for example), as further described below.

In some embodiments, the frame 12 can be without posts 24 and apertures 26 can be formed in the apices 18 at the outflow end 22 of the frame 12. In the embodiment shown at FIG. 3, the apertures are formed at the outflow end of the frame so that when loaded within the delivery apparatus 100, a releasable connection can be formed between a cord manifold 120 and the outflow end 22 of the frame 12 via cords 118, as described further below. This arrangement facilitates delivery of the prosthetic valve 10 to the native aortic valve using a retrograde delivery approach whereby the delivery apparatus 100 is advanced through a femoral artery and the aorta to access the native aortic valve.

In other embodiments, the apertures 26 (whether formed in posts 24 or in the apices 18) can be formed at the inlet (or inflow) end 20 of the frame 12 where other delivery apparatus configurations or other delivery techniques require apertures at the inlet end of the frame, such as a transapical delivery approach. In still further embodiments, the delivery apparatus 100 can include the cord manifold 120 positioned distal to the prosthetic valve when loaded within the delivery apparatus, with the cord manifold coupled to the inlet (or inflow) end 20 of the frame.

In particular embodiments, the prosthetic heart valve 10 is a self-expandable heart valve wherein the frame 12 is a made of a super-elastic, self-expanding material (e.g., a nickel titanium alloy such as Nitinol) as is known in the art. When used with the delivery apparatus 100 (FIG. 2), the prosthetic valve 10 can self-expand from a radially compressed state to a radially expanded state when advanced from a delivery capsule (e.g., a delivery sheath) of the delivery apparatus.

In other embodiments, the frame 12 can be made of any of various suitable plastically-expandable materials (e.g., stainless steel, cobalt-chromium alloy, etc.) and the prosthetic heart valve can be expanded from a radially compressed state to a radially expanded state by inflating a balloon of the delivery apparatus or by actuating other expansion means of the delivery apparatus and produces radial expansion of the prosthetic valve.

The valvular structure 14 can comprise a plurality of leaflets 28. The valvular structure typically comprises three leaflets 28 arranged in a tricuspid arrangement, although a greater or fewer number of leaflets 28 can be used. The leaflets 28 can be made of any various suitable materials, including natural tissue (e.g., bovine pericardium or pericardium from other sources) or synthetic materials (e.g., polyurethane). Adjacent side portions at the outflow edges (the upper edges in the drawings) of adjacent leaflets can be secured to each other to form commissures 30 of the valvular structure, which can be secured to the frame with sutures 32.

The prosthetic valve 10 can further include an inner skirt 34 mounted on the inside of the frame 12. The skirt 34 helps establish a seal with the surrounding tissue after implantation. The skirt 34 can also be used to mount portions of the leaflets 28 to the frame 12. For example, in the illustrated embodiment, the inflow edges of the leaflets (the lower edges in the drawings) can be sutured to the skirt 34 along suture line 36. The skirt 34 can be connected directly to the frame 12, such as with sutures. Although not shown, the prosthetic valve 10 can include an outer skirt mounted on the outside of the frame in lieu of or in addition to the inner skirt 34 to further seal the prosthetic valve against surrounding tissue. The inner and/or outer skirts can be made of any of various suitable materials, including natural tissue (e.g., pericardium tissue) or any of various synthetic materials, which may be woven, non-woven, braided, knitted, and/or combinations thereof. In one specific implementation, the inner skirt 34 is made of a polyethylene terephthalate (PET) fabric.

Exemplary configurations of the prosthetic heart valve are further disclosed in U.S. Patent Application Publication Nos. 2014/0343670, 2012/0123529, 2010/0036484, and 2010/0049313.

Prosthetic heart valve 10, or another type of implantable, expandable medical device, such as an expandable stent, can be delivered to an implantation site via a delivery apparatus, an embodiment of which is shown at FIGS. 2.

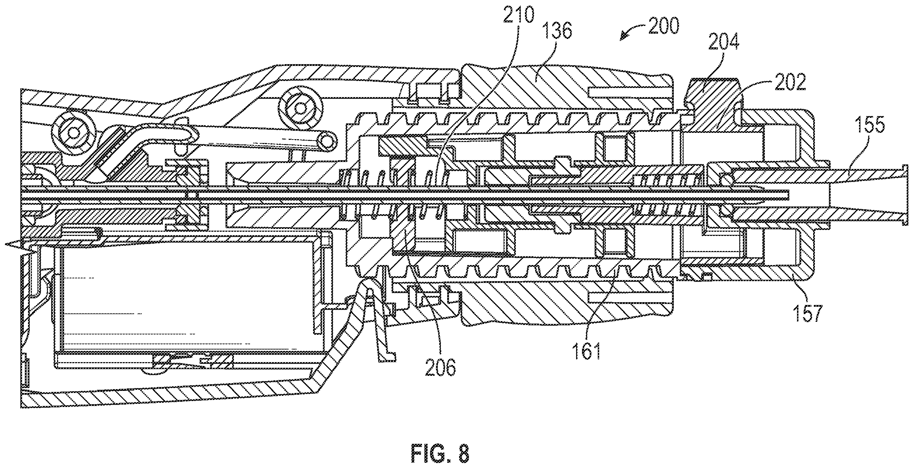

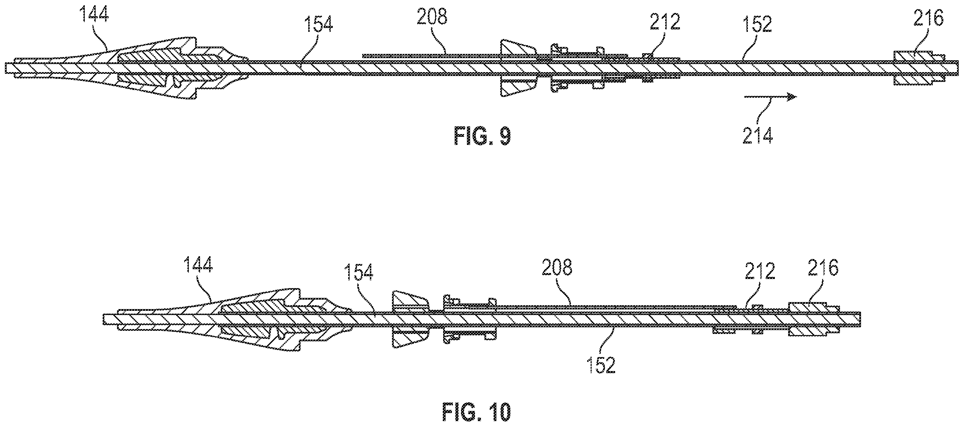

FIGS. 2-7 show an exemplary embodiment of a delivery apparatus 100 which can be used to delivery a prosthetic medical device, such as prosthetic heart valve 10 shown in FIG. 1, to a target implantation site in a patient. In some embodiments, a handle portion 132 of the delivery apparatus 100 can include a locking mechanism for a release mechanism of a release assembly of the delivery apparatus 100, as shown in FIG. 8. An exemplary distal portion of the release assembly, which may be used in delivery apparatus 100, is shown in FIGS. 9 and 10.

As shown in FIG. 2, the delivery apparatus 100 can comprise a handle portion 132 and a first shaft 134 extending distally therefrom. A user, such as a physician or clinician, can operate the delivery apparatus 100 via actuation of a plurality of knobs 136, dials, and/or buttons 138a, 138b located on the handle portion 132. The first shaft 134 has a proximal end portion 140 and a distal end portion 142. The proximal end portion 140 of the first shaft 134 can be coupled to the handle portion 132. The handle portion 132 can comprise a housing 133. In some embodiments, the housing 133 can include two housing portions.

As shown in FIG. 3, the delivery apparatus 100 can include a second shaft 150 and a third shaft 152. The second shaft 150 extends distally from the handle portion 132 and co-axially through the first shaft 134. The third shaft 152 extends distally from the handle portion 132 and co-axially though the second shaft 150. In the illustrated, the first shaft 134 is the outermost shaft of the delivery apparatus 100 and therefore can be referred to as the outer shaft 134 of the delivery apparatus 100. In the illustrated embodiment, the third shaft 152 is the innermost shaft of the delivery apparatus and therefore can be referred to as the inner shaft 152 of the delivery apparatus 100. In the illustrated embodiment, the second shaft 150 is intermediate to, or between, the innermost shaft and the outermost shaft and therefore can be referred to as an intermediate shaft.

A nosecone 144 can be connected to or mounted on a distal end portion 152d of the inner shaft 152. The nosecone 144 can have a tapered outer surface as shown for atraumatic tracking of the delivery apparatus 100 through a patient's vasculature. The inner shaft 152 extends distally beyond the intermediate shaft 150, through a lumen of a cord manifold 120, and through the prosthetic valve 10.

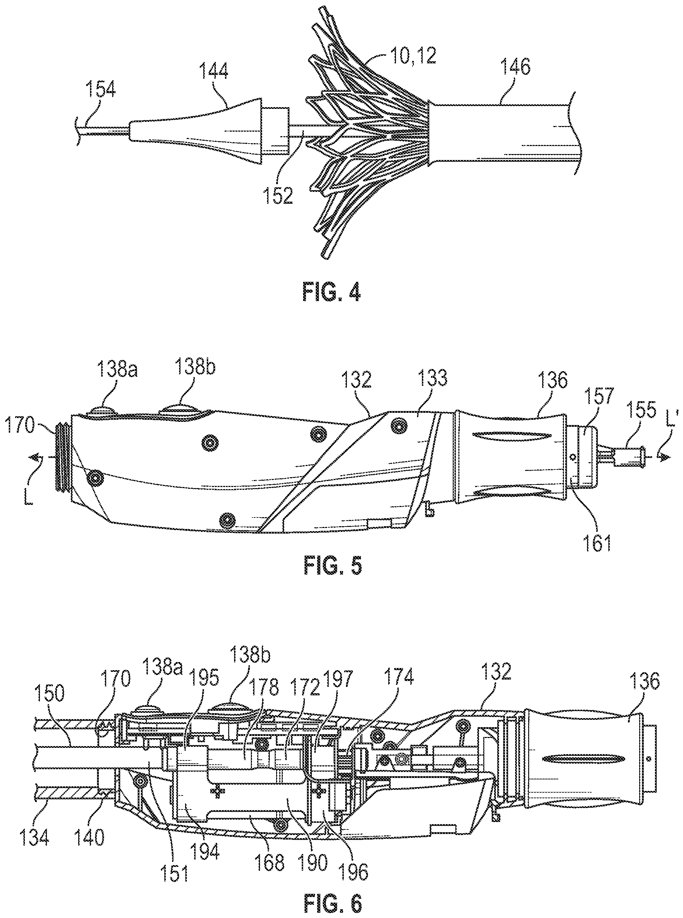

In certain embodiments, the first, second, and third shafts 134, 150, and 152, respectively, can be configured to be moveable relative to each other, including relative axial movement (in the proximal and distal directions) and/or relative rotational movement (in the clockwise and counterclockwise directions). A guide wire 154 (FIG. 4) can extend through the central lumen of the inner shaft 152 and the inner lumen of the nosecone 144 so that the delivery apparatus 100 can be advanced over the guide wire 154 inside the patient's vasculature during delivery of the prosthetic valve 10 to a target implantation site. The guide wire 154 may exit the inner shaft 152 via a proximal port 155 of a cap 157 of the handle portion 132 (FIG. 5). As shown in FIG. 5 and FIG. 8, the cap 157 can be coupled to an end of a drive screw 161 of a release mechanism 200 of the delivery apparatus 100 (as described further below with reference to FIGS. 8-10).

A delivery capsule 146 is coupled to the distal end portion 142 of the first shaft 134, proximal to the nosecone 144. The delivery capsule 146 houses the prosthetic valve 10 therein in a radially compressed state, as shown at FIGS. 3-4. In one embodiment, the delivery capsule 146 covers and retains the underlying compressed prosthetic valve of FIG. 1. The delivery apparatus 100 is particularly suited for delivering and implanting a self-expandable prosthetic valve 10 that radially expands to its functional size under its own resiliency when deployed from the delivery capsule 146.

However, the prosthetic heart valve 10 alternatively can be a plastically expandable prosthetic valve or a mechanically expandable heart valve. If the delivery apparatus is used to implant a plastically expandable valve, the delivery apparatus can include a balloon catheter as known in the art for expanding the prosthetic valve, such as disclosed in U.S. Publication No. 2009/0281619. If the delivery apparatus is used to implant a mechanically expandable valve, the delivery apparatus can include one or more actuators for expanding the prosthetic valve, such as disclosed in International Application No. PCT/US2020/063104.

As shown at FIG. 3, the delivery capsule 146 is configured to accommodate the prosthetic heart valve 10, or another type of implantable medical device, in a radially compressed state for delivery into a patient's vasculature. The cord manifold 120 is configured to form a releasable connection with the prosthetic heart valve 10 via a plurality of cords or tethers 118 (FIG. 3). The cord manifold 120 is coupled to a distal end of the first shaft 134, proximal to each of the nosecone 144 and the crimped prosthetic valve 10.

As shown in FIG. 3, the cord manifold 120 can include a proximal portion 122 and a distal portion 124, the distal portion 124 spaced axially away from the proximal portion 122. The proximal portion 122 of the cord manifold 120 can be fixedly secured to the distal end portion 142 of the first shaft 134 using suitable techniques or mechanisms, such as via mechanical connectors, welding, a press-fit, and/or an adhesive. For example, in some embodiments, the distal end portion 142 of the shaft 134 can extend into a lumen of the proximal portion 122, which can be secured to the shaft 134 using any of the connection techniques described above.

The cords 118 may be made of any of various suitable biocompatible materials for use within a patient's body. In certain embodiments, a cord 118 can comprise a single filament cord or a multifilament or multi-strand cord formed from braiding, weaving, knitting, twisting, and wrapping a plurality of filaments or strands together. The filaments or strands can comprise polymeric fibers, such as ultra-high molecular weight polyethylene, nylon, polyester, and/or aramid, or flexible wires (e.g., metal wires).

Each of the cords 118 can have a first end 118a attached to the cord manifold 120, such as to the proximal portion 122. Each cord 118 extends through an opening of the frame 12 of the prosthetic valve (e.g., through an opening 26) and can have a second end 118b in the form of a loop that is retained on a release member 156. The release members 156 are configured to retain the cords 118 in a state connected to the frame 12 of the prosthetic valve 10 until they are actuated by a user to release the cords 118. Two release members 156 are shown for purposes of illustration. It should be understood that any number of release members 156 can be used.

Similarly, two cords 118 are shown for purposes of illustration, but it should be understood that any number of cords can be used. Also, there need not be an equal number of cords and release members 156. For example, the ends 118b of multiple cords 118 may be retained on a single release member 156. Desirably, at least three cords 118 are used to balance the attachment of the frame 12 to the cord manifold 120. In particular embodiments, the number of cords 118 is equal to the number of apices 18 of the frame 12 of the prosthetic valve 10 (FIG. 1). Moreover, in other embodiments, a single cord can be used to connect the frame 12 to the cord manifold 120 at multiple locations along the outflow end of the frame by forming multiple passes extending through openings of the frame.

Each release member 156 can extend in a slideable manner through respective openings in the proximal portion 122 and the distal portion 124 of the cord manifold 120 (FIG. 3). In some embodiments, each release member 156 can extend through the first shaft 134, along its entire length, and can have proximal end portions that are operatively coupled to a knob 136 on the handle portion 132 to control movement of the release members 156. In alternate embodiments, each release member 156 can have a proximal end that is coupled to an outer surface of the third shaft 152 of the delivery apparatus 100.

Each of the release members 156 is moveable in the proximal and distal directions relative to the proximal and distal portions 122, 124, of the cord manifold between a distal position where each release member 156 retains a respective cord 118 and a proximal position where each release member 156 is released from a respective cord 118. Together, the knob 136, the release members 156, and the cord manifold 120 can form a release assembly of the delivery apparatus 100, as described further below with reference to FIG. 8 (e.g., the release mechanism 200 of the release assembly, arranged within the handle portion 132, is shown in FIG. 8).

Further details regarding the attachment of the prosthetic valve 10 to the delivery apparatus 100 via one or more cords or sutures are disclosed in U.S. Publication Nos. 2014/0343670, 2012/0239142, and 2010/0049313 and International Application No. PCT/US2020/024130.

Moreover, in alternative embodiments, different valve-retaining mechanisms can be used to form a releasable connection between the prosthetic valve 10 and the delivery apparatus 100. For example, in some embodiments, the posts 24 of the frame 12 can be retained in corresponding recesses of a shaft or retaining member of the delivery apparatus, which allow the posts of the frame to expand out of their corresponding recesses when the capsule 146 is retracted to deploy the prosthetic valve. In other embodiments, the retaining mechanism can comprise inner and outer metal fork members that form a release connection between the delivery apparatus and the prosthetic valve. Further details regarding alternative valve-retaining mechanisms are disclosed in U.S. Publication Nos. 2012/0239142 and 2010/0049313.

As further shown in FIG. 3, the second shaft 150 can include an externally threaded portion 162 along a distal end portion thereof. The threaded portion 162 can comprise threads formed on the external surface of the shaft or can be a separate screw connected to the distal end of a proximal shaft section. The capsule 146 is operatively connected to the second shaft 150 by an internally threaded nut 164 disposed on the threaded portion 162. The nut 164 can have a radially extending projection 166 that extends into a corresponding opening in the capsule 146 (see FIG. 2). Rotation of the nut 164 is restricted by one or more rails 165 extending from or formed along the distal end portion of the first shaft 134.

Rotation of the second shaft 150 relative to the first shaft 134 therefore produces axial movement of the nut 164 (in the distal and proximal directions), which in turn produces corresponding axial movement of the capsule 146 in the same direction during loading, deployment, and/or recapture of the prosthetic valve. For example, when the nut 164 is in a distal position, the delivery capsule 146 extends over and retains the prosthetic valve 10 in a compressed state for delivery. Movement of the nut 164 in a proximal direction causes the delivery capsule 146 to move in the proximal direction, thereby deploying the prosthetic valve. Rotation of the second shaft 150 can be achieved via a motor operatively coupled to the second shaft and/or manual control features, as further described below.

In certain embodiments, the delivery apparatus 100 may comprise one or more steering mechanisms configured to control the curvature of one or more of the shafts 134, 150, 152 to assist in steering the delivery apparatus through a patient's vasculature. For example, the steering mechanism can comprise one or more eccentrically positioned pull wires extending through a shaft and operatively connected to an adjustment mechanism, such as steering knob 418, located on or adjacent the handle portion 132 FIG. 2). Adjustment of the adjustment mechanism is effective to change the tension of the pull wires to cause the shaft to curve in a given direction, or to straighten. In one implementation, one or more pull wires extend though the outer shaft 134 and adjustment of the adjustment mechanism is effective to adjust the curvature of the distal end portion of the outer shaft 134 and the delivery apparatus 100. Further details regarding the steering mechanism are disclosed in U.S. Publication Nos. 2007/0005131 and 2013/0030519.

In certain embodiments, as shown in FIGS. 6-8, the delivery apparatus 100 is a motorized apparatus comprising a motor 168 housed inside the handle portion 132. The motorized embodiment automates deployment of the prosthetic valve 10. In particular, the motor 168 is operatively coupled to the second shaft 150 to produce rotation of the second shaft 150 relative to the first shaft 134 and corresponding axial movement of the capsule 146, as further described below.

The proximal end portion 140 of the first shaft 134 can be coupled to a distal end of the handle portion 132. As shown at FIG. 6, a proximal end portion 151 of the second shaft 150 can extend into the handle portion 132 via a distal opening 170 of the handle portion 132. A rotatable component 172 (which can be referred to as a drive cylinder in some embodiments) is disposed inside the handle portion 132 and operatively coupled to the second shaft 150.

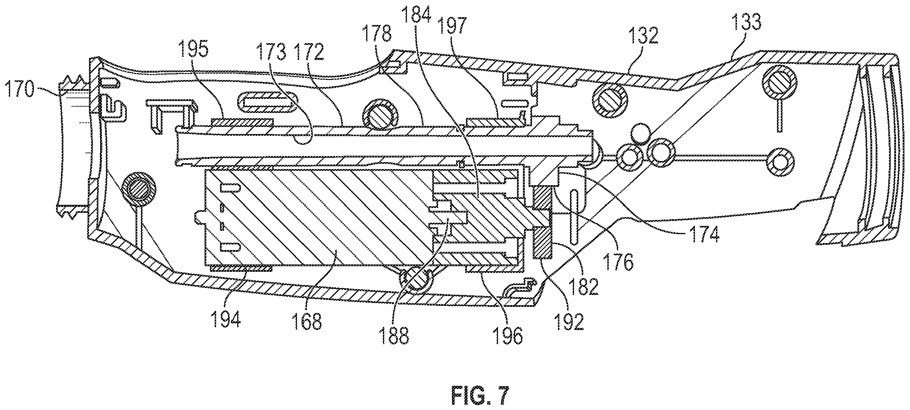

In one embodiment, as best shown at FIG. 7, the proximal end portion of the rotatable component 172 comprises a gear 174 having a plurality of gear teeth 176 that are circumferentially arrayed relative to each other. The rotatable component 172 further comprises a main body 178 configured as an extended shaft having a lumen 173. In the illustrated embodiment the main body 178 and gear 174 are integrally formed, although they may be separately formed components that are connected to each with any of various attachment means. The main body 178 of the rotatable component 172 can be coaxial to a central longitudinal axis L-L' (shown in FIG. 5) of the handle portion 132 and also coaxial to the first shaft 134. The lumen 173 of the main body 178 can be sized to receive and hold the proximal end portion 151 of the second shaft 150 therein.

In some implementations, the inner surface of the lumen 173 can have a non-circular cross-section in a plane perpendicular to the longitudinal axis L-L' and the proximal end portion 151 of the second shaft 150 can have a similar cross-sectional profile that corresponds to the shape of the lumen so that rotational motion of the rotatable component 172 is transferred to the second shaft 150. For example, the lumen 173 and the proximal end portion 151 can be generally cylindrical have a series of circumferentially spaced flat sections. In lieu of or in addition to providing the lumen 173 and proximal end portion 151 with non-circular cross-sections, the proximal end portion 151 can be secured to the rotatable component with fastening means, such as mechanical fasteners (e.g., a screw), adhesives, a press fit, a snap fit connection, etc.

As best shown in FIG. 6, the motor 168 may be held inside a retaining case or cradle 190. The motor can be an electric motor and the handle portion can include a battery compartment that contains one or more batteries (not shown) for powering the motor 168. One or more operator buttons 138a, 138b on the handle portion 132 allow a user to activate the motor 168, such as by electrically coupling current from the battery power to the motor. The motor can be rotated in either direction, as described below, thereby moving the capsule 146 in either the proximal or distal direction. One of the buttons (e.g., button 138a) can be operable to rotate the motor in a first rotational direction to move the capsule 146 in a distal direction, such as for loading the prosthetic valve in the capsule 146, and the other button (e.g., button 138b) can be operable to rotate the motor 168 in a second rotational direction to move the capsule 146 in a proximal direction, such as for deploying the prosthetic valve. In lieu of or in addition to one or more batteries, the motor 168 can be configured to receive a power cord that supplies electric current to the motor from a power source external to the handle portion 132 (e.g., a wall outlet).

As best shown in FIG. 7, the motor 168 can be coupled to the rotatable component 172 by a drive shaft 184 connected to a motor shaft 188 and an intermediate drive gear 182 connected to the drive shaft 184. The drive gear 182 can have circumferentially arrayed gear teeth 192 that can engage with the circumferentially arranged gear teeth 176 of the rotatable component 172. When driven by the motor, the motor 168 rotates the motor shaft 188, which in turn rotates the drive shaft 184 and the drive gear 182. The drive gear 182 engages and rotates the gear 174 of the rotatable component 172, thereby rotating the rotatable component 172 and the second shaft 150. The drive gear 182 can be positioned radially offset from a central axis of the rotatable component 172 and a central longitudinal axis L-L' of the handle portion 132 such that when meshed, the gears are vertically aligned. In further embodiments, one or more additional gears may be provided between the drive gear 182 and the rotatable component 172 to transfer rotation from the motor to the rotatable component.

In alternative embodiments, the motor shaft 188 or the drive shaft 184 can be connected to the rotatable component 172 without any intervening gears. For example, the motor shaft 188 can be positioned proximal to the rotatable component along the axis L-L' and the motor shaft 188 can be connected to the rotatable component 172 in a direct drive arrangement.

In the illustrated embodiment, the cradle 190 housing the motor 168 and the drive shaft 184 may also be configured to support the rotatable component 172 for rotational movement within the handle portion. As best shown in FIG. 6, the cradle 190 may have a first distal portion 194 that includes a distal sleeve 195 encircling a distal end portion of the main body 178 of the rotatable component 172. The cradle 190 may further have proximal portion 196 that includes a proximal sleeve 197 encircling a proximal end portion of the main body 178 of the rotatable component 172.

With reference to FIGS. 3 and 7, rotation of the motor 168 in a first direction (e.g., clockwise or counterclockwise) causes rotation of the rotatable component 172. This in turn causes rotation of the second shaft 150, which is coupled to the rotatable component 172. Rotation of the second shaft 150 causes rotation of the threaded portion (screw) 162 of the second shaft 150. As described above, rotation of the threaded portion 162 produces axial movement of the drive nut 164 and the capsule 146 (FIG. 3). For example, rotation of the rotatable component in the first direction can cause the delivery capsule 146 to retract in a proximal direction and uncover the prosthetic valve at the distal end of the delivery apparatus 100. Conversely, rotation of the motor in a second direction, opposite to the first direction, causes the second shaft 150 to rotate in an opposite direction, which causes the nut to move axially in the opposite direction, moving the delivery capsule 146 in a distal direction back over the prosthetic valve. An operator can actuate buttons 138a, 138b (FIGS. 2 and 6) on the handle portion 132 to activate the motor 168, and axially move the delivery capsule 146 in a motorized manner. This allows for quick deployment or retrieval of the prosthetic valve 10.

In use, the prosthetic valve 10 can be connected to the delivery apparatus 100 and loaded into the capsule 146 as follows. A releasable connection can be formed between each apex 18 at one end of the frame 12 and the cord manifold 120 with a separate cord 118. Optionally, the length of the cords 118 are selected such that the secured end of the frame is held in an at least partially radially compressed state by the cords. After securing the end of the frame 12 with the cords 118, the delivery capsule 146 can be advanced distally (e.g., by pressing button 138a) over the cord manifold 120, the cords 118, and the frame 12, causing the frame to collapse to a radially compressed state under the force of the capsule 146 (as shown in FIG. 4). The delivery capsule 146 is advanced distally until the distal end of delivery capsule 146 abuts the nosecone 144 to fully enclose the prosthetic valve 10, as shown in FIG. 3.

After loading the prosthetic heart valve 10 within the delivery apparatus 100, as described above, the delivery apparatus 100 can be inserted in the vasculature of a patient and advanced or navigated through the patient's vasculature to the desired implantation site (e.g., through a femoral artery and the aorta when delivering the prosthetic valve 10 in a retrograde delivery approach to the native aortic valve).

Once the prosthetic valve 10 is delivered to a selected implantation site within the patient (e.g., the native aortic valve), the delivery capsule 146 may be retracted (e.g., by pressing button 138b) in order to deploy the prosthetic valve 10. As the delivery capsule 146 is retracted (FIG. 4), the prosthetic valve 10 can radially self-expand under the resiliency of the frame 12. After the delivery capsule 146 is fully retracted from the prosthetic valve 10, the prosthetic valve is still attached to the delivery apparatus 12 by the cords 118. While still attached to the delivery apparatus 100, the user can manipulate the delivery apparatus (e.g., by moving it in the proximal and distal directions and/or rotating it) to adjust the position of the prosthetic valve 10 relative to the desired implantation location.

If desired, the delivery capsule 146 can be advanced back over the prosthetic valve 10 to fully or partially recapture the prosthetic valve (bring the prosthetic valve back within the capsule) to facilitate re-positioning of the prosthetic valve. For example, after crossing the native aortic valve leaflets in a retrograde delivery approach and deploying the prosthetic valve, it may be desirable to recapture the prosthetic valve back within the capsule 146, retract the delivery apparatus 100 to bring the prosthetic valve back within the aorta, and then advance the prosthetic valve back across the native aortic valve leaflets, and deploy the prosthetic valve from the capsule.

Once the prosthetic valve is deployed from the capsule 146 and positioned at the desired implantation location, the release members 156 can be retracted, such as by rotating the knob 136 on the handle portion 132. In some cases, the cords 118 slide outwardly from the apertures 26 and free themselves from the frame 12 by virtue of the self-expanding frame 12 further expanding when the release members 156 are retracted. In other cases, the user can slightly retract the delivery apparatus 100, which in turn pulls the cords 118 proximally relative to the frame 12 to pull them out of the apertures 26.

Optionally, the orientation of the prosthetic valve can be reversed such that the inflow end of the prosthetic valve is the proximal end and the outflow end of the prosthetic valve is the distal end when coupled to the delivery apparatus. This can facilitate delivery of the prosthetic valve to different implantation locations (e.g., the native aortic, pulmonary, mitral, and tricuspid annuluses) and/or for various delivery approaches (e.g. antegrade, transseptal, trans ventricular, transatrial). Further details on the components and operation of delivery apparatuses used to deliver prosthetic medical devices, such as a prosthetic heart valve, to a target location are disclosed in International Patent Application No. PCT/US2021/023696.

FIG. 8 shows a portion of an embodiment of the handle portion 132 of delivery apparatus 100 which includes a release mechanism 200, the release mechanism 200 including the knob 136, the drive screw 161, and a locking mechanism 202. The release mechanism 200 is configured to facilitate the release of the prosthetic heart valve from the delivery apparatus, via control of release members 208, which, in some embodiments, may be the same or similar to release members 156, as described above with reference to FIG. 3. Together, the release mechanism 200 and the release members 208 can form a release assembly of the delivery apparatus 100. FIGS. 9 and 10 show an embodiment of a distal portion of the release assembly of the delivery apparatus 100, including release members 208.

As introduced above, the delivery apparatus 100 can include an inner shaft 152 having an inner, guide wire lumen configured to receive a guide wire (e.g., guide wire 154) therein. The inner shaft 152 can be releasably connected at its proximal end to the drive screw 161 (FIG. 8) and connected at its distal end to valve release members 208 (FIGS. 9 and 10). Thus, as described further below, axial movement of the inner shaft 152 can result in axial movement of the release member 208 (FIGS. 9 and 10).

As shown in FIG. 8, the release mechanism 200 can include the drive screw 161 arranged within and interfacing with the knob (release knob) 136. The cap 157 is coupled to a proximal end of the drive screw 161. The locking mechanism 202 can be coupled to the proximal end of the drive screw 161 and arranged within a portion of the cap 157. The locking mechanism 202 can be rotatable, via knob 204 (which may be manually actuated by a user). For example, rotation of the knob 204, and thus the locking mechanism 202, can cause a washer 206 to clamp onto (locked position) and off (unlocked position) an outer surface of the inner shaft 152. The washer 206 can be fixedly coupled to the drive screw 161, and as a result, can move axially with axial translation of the drive screw 161. Thus, when the release mechanism 200 is locked to the inner shaft 152, rotation of the knob 136, which causes axial translation of the drive screw 161, causes axial translation of the inner shaft 152.

As shown in FIGS. 9 and 10, the inner shaft 152 can extend to and/or into the nosecone 144. A spool 212 can be coupled (e.g., fixedly coupled) to a portion of the outer surface of the inner shaft 152 and proximal ends of the release members 208 can be coupled to the spool 212. As described above with reference to FIG. 3, distal ends of the release members 208 can be removably coupled to coupling elements (e.g., cords, tethers, sutures, or the like) coupled to the prosthetic heart valve.

FIG. 9 shows the distal portion of the release assembly in a starting configuration, prior to releasing a prosthetic heart valve from the delivery apparatus 100. For purposes of illustration, the prosthetic heart valve is not shown in FIGS. 9 and 10. However, in FIG. 9, the distal ends of the release members 208 may be in a position that couples the valve to the delivery apparatus 100 (e.g., prevents the valve from moving axially relative to the delivery apparatus 100). In this configuration, the locking mechanism 202 can be in a locked state.

After the distal end portion of the delivery apparatus 100, containing the prosthetic heart valve, reaches the target implantation site, the prosthetic heart valve may be deployed by moving the capsule 146 away from the valve, to uncover the valve. The knob 136 can then be rotated in order to move the inner shaft 152 axially, in a proximal direction 214 (toward the handle portion 132), thereby retracting the release members 208 away from the prosthetic heart valve.

As used herein "proximal direction" may refer to a direction of movement or travel, along an axial direction that is parallel to the central longitudinal axis of the delivery apparatus, toward the handle portion or a user of the delivery apparatus while a "distal direction" may refer to a direction of movement or travel, opposite to the proximal direction along the axial direction, that is away from the handle portion and closer to the target implantation site.

FIG. 10 shows the distal portion of the release assembly in a retracted (e.g., released) configuration, after releasing the prosthetic heart valve from the delivery apparatus 100. In this configuration, the inner shaft 152 has translated proximally, thereby translating the spool 212 proximally. In some embodiments, as shown in FIG. 10, the spool 212 may come into contact with a spool stop 216 which prevents further axial movement of the spool 212 and release members 208 in the proximal direction 214.

The spool stop 216 can be fixed axially relative to the inner shaft 152 and the spool 212. In some embodiments, the spool stop 216 can be fixed to a component of another shaft of the delivery apparatus, such as second shaft 150 or first shaft 134.

Since the release members 208 have also moved proximally, with the spool 212, the prosthetic heart valve can now be released from the delivery apparatus 100 and the delivery apparatus 100 can be removed from the implantation site.

In some embodiments, when delivering a prosthetic aortic valve with a delivery apparatus (e.g., delivery apparatus 100) to its target implantation site, at least the distal end portion of the delivery apparatus may have to traverse a curved portion of the patient's vasculature, such as the aortic arch of the patient. An exemplary, simulated aortic arch 300 is shown in FIG. 11. Thus, as introduced above, in some embodiments, the delivery apparatus can include one or more steering mechanisms configured to control the curvature of one or more of the shafts of the delivery apparatus 100 (e.g., shafts 134, 150, 152, and/or 152) to assist in steering the delivery apparatus through a patient's vasculature.

For example, as shown in FIG. 11, the one or more steering mechanisms may allow at least the distal end portion 302 of the delivery apparatus 304 (which may be the same or similar to delivery apparatus 100) to flex and articulate (e.g., curve) around the aortic arch 300. When the distal end portion 302 of the delivery apparatus 304 articulates around the aortic arch, the distal end portions of the concentric shafts (e.g., shafts 134, 150, 152, and/or 152) of the delivery apparatus 304 may shorten or lengthen relative to one another. In some embodiments, after flexing the distal end portion 302 of the delivery apparatus 304, to reach the target implantation site, and while the distal end portion 302 remains flexed (as shown in FIG. 11), the valve can be released from the delivery apparatus 304 by rotating the knob 136 of the release mechanism 200. This causes linear movement (axially, in the proximal direction) of the drive screw 161, the inner shaft 152, and the release members 208. However, this linear translation of concentric lumens when flexed can cause shortening of the inner shaft 152, thereby creating tension in the distal end portion 302 upon unflexing. In this state, the inner shaft 152 can become a tensioned pull wire. If the release mechanism 200 is not unlocked (e.g., to relieve this tension) during unflexing, the distal end portion 302 is held in tension, thereby preventing the delivery apparatus from being removed from the implantation site.

In this way, the locking mechanism of the release mechanism 200 may increase the complexity of an implantation procedure and cause tension issues that may increase the difficulty in removing the delivery apparatus from the implantation site, after implanting the prosthetic heart valve. Thus, it may be desirable to have a delivery apparatus that does not include a locking mechanism for the release mechanism.

FIGS. 12-33 show an embodiment of a release mechanism 402 for a handle portion 400 of a delivery apparatus. In some embodiments, the handle portion 400 may replace the handle portion 132 of delivery apparatus 100 shown in FIG. 8. In some embodiments, the handle portion 400 may control operation of a distal end portion of the delivery apparatus, such as the distal end portion shown in FIGS. 9 and 10. Further, the delivery apparatus can be configured to deliver a radially compressed prosthetic medical device, such as the prosthetic heart valve 10 of FIG. 1, arranged on a distal end of the distal end portion of the delivery apparatus, to a target implantation site.

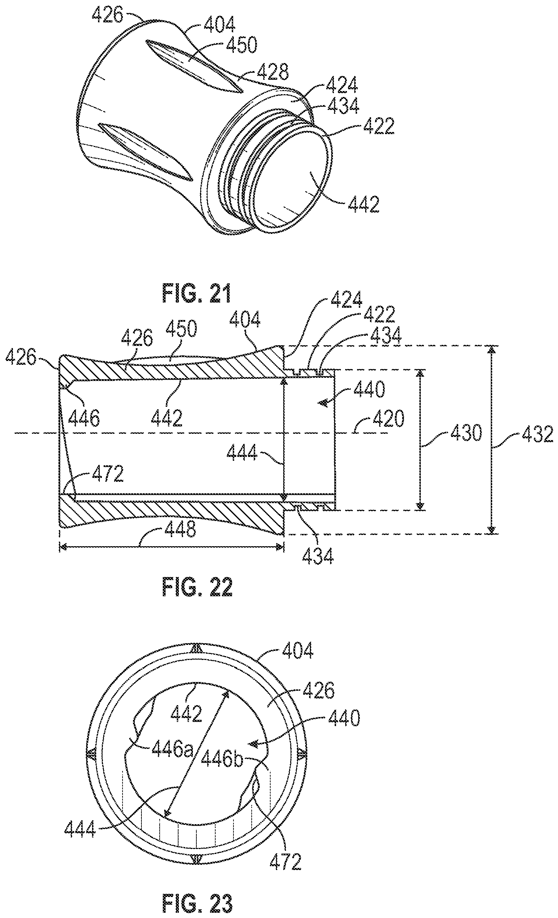

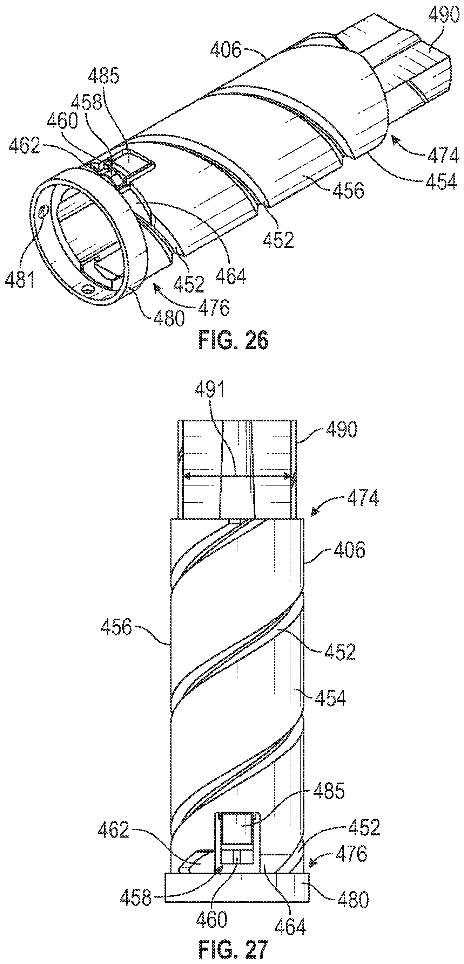

The release mechanism 402 of FIGS. 12-33 is configured to automatically provide tension relief of tension created by flexing a distal end portion of the delivery apparatus and releasing the radially compressed medical device mounted on the distal end portion of the delivery apparatus, as explained above. As such, the release mechanism 402 does not include a locking mechanism (e.g., locking mechanism 202 of FIG. 8). FIGS. 12-20 show the assembled handle portion 400 including the release mechanism 402 in different positions or configurations during a prosthetic medical device implantation procedure with the delivery apparatus. FIGS. 21-25 show different views of a release knob 404 of the release mechanism 402 (disassembled from a remainder of the handle portion) and FIGS. 26-33 show different views of a drive screw 406 of the release mechanism 402 (disassembled from the remainder of the handle portion).

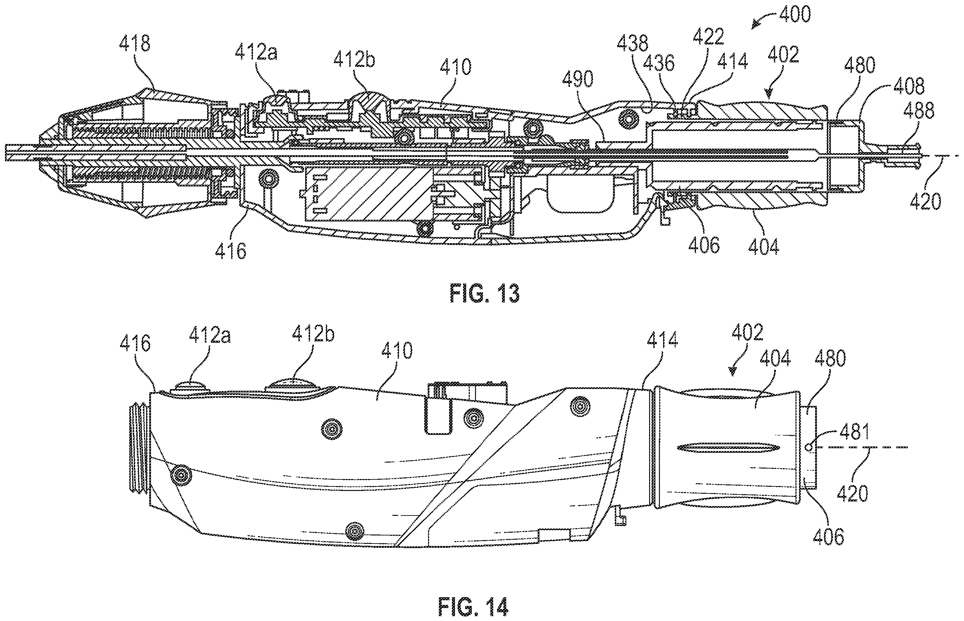

As shown in FIGS. 12-18 and 20, the handle portion 400 can include a housing 410 (e.g., outer housing). The housing 410 can house internal components of the handle portion 400, such as those described above with reference to FIGS. 2-8 and further below with reference to FIGS. 13, 15, 17, and 18. In some embodiments, the handle portion 400 can include a plurality of knobs and buttons that are actuatable by a user (e.g., physician or clinician) in order to control operation of the delivery apparatus. For example, in some embodiments, the handle portion 400 can include buttons 412a and 412b, which may be similar to buttons 138a and 138b, as described above with reference to FIGS. 2, 5, and 6.

In some embodiments, as shown in FIGS. 12 and 13, a steering knob 418 of a steering mechanism of the delivery apparatus can be coupled to a distal end 416 of the housing 410. The steering mechanism, via adjustment (e.g., rotation) of the steering knob 418, can be configured to adjust a curvature or amount of flexing of one or more shafts of the delivery apparatus, at a distal end portion of the delivery apparatus, as described previously herein.

The release knob 404 of the release mechanism 402 can be coupled to a proximal end 414 of the housing 410 and configured to rotate, about a central longitudinal axis 420 of the release mechanism (which may also be a central longitudinal axis of the knob 404, drive screw 406, and delivery apparatus). However, movement of the release knob 404 may be fixed in the axial direction (along central longitudinal axis 420). In this way, the release knob 404 can rotate but may be fixed from translating linearly, in the axial direction.

For example, as shown in FIGS. 21, 22, and 24, the release knob 404 can include a main body 428 having a distal end 424 and a proximal end 426. The body 428 may be a portion of the release knob 404 that is configured to be held and rotated by a user. The release knob 404 can further include a collar 422 that extends outward, in the axial direction, from the distal end 424 of the release knob 404. An outer diameter 430 of the collar 422 can be smaller than an outer diameter (e.g., outermost diameter of a widest portion) 432 of the body 428, as shown in FIG. 22. The collar 422 can include one or more grooves (or channels) 434 extending around a circumference (e.g., entire circumference) and indented into an outer surface of the collar 422. As shown in FIGS. 21, 22, and 24, the collar 422 includes two grooves 434 spaced apart from one another on the collar 422, in the axial direction. However, in alternate embodiments, the collar 422 can include more or less that two grooves 434 (e.g., one, three, or the like). Each groove 434 is configured (e.g., shaped) to mate with a corresponding annular protrusion 436 of the housing 410.

For example, as shown in FIGS. 13, 15, 17, and 18, the inner surface 438 of the housing 410, at its proximal end 414, includes one or more annular protrusions 436, each extending from an inner surface 438 of the housing 410, radially inward toward the central longitudinal axis 420. The collar 422 can extend into an interior of the proximal end 414 of the housing 410. Each of the annular protrusions 436 of the collar 422 can extend around an entire circumference of the inner surface 438. The number of annular protrusions 436 can match a number of the grooves 434. In this way, each annular protrusion 436 can extend into and mate with a corresponding groove 434. There may be enough clearance between a mated annular protrusion 436 and groove 434 to allow the release knob 404 to rotate, while the housing 410 remains fixed from rotating, and also prevent the release knob 404 from moving axially relative to the housing 410. In this way, the release knob 404 is configured to rotate but is fixed from linear translation (in the axial direction and the radial direction), relative to the housing 410, due to the mating connection between the grooves 434 and the annular protrusions 436 of the housing 410.

Returning to FIGS. 21-25, the release knob 404 can include an internal, cylindrical bore (or cavity) 440 defined by an inner surface 442 of the release knob 404 that extends from the proximal end 426 of the body 428 to a distal end of the collar 422. The inner surface 442 can define an inner diameter 444 of the release knob 404 (FIG. 22). The bore 440 is configured to receive the drive screw 406 therein, as shown in FIGS. 13, 15, 17, and 18. The drive screw 406 and the release knob 404 can be coaxial with one another (e.g., the central longitudinal axis 420 may be the common axis).

As shown in FIGS. 22-25, the release knob 404 can include one or more teeth 446 extending in a radial direction, toward the central longitudinal axis 420, from the inner surface 442. Each tooth 446 is arranged at the proximal end 426 of the release knob 404. For example, each tooth 446 can extend along the inner surface 442, from the proximal end 426, toward the distal end 424 of the release knob 404, only a portion of a total distance (length) 448 between the proximal end 426 and the distal end 424. In the presently claimed invention, the portion is less than ¼ the total distance 448. In some embodiments of the presently claimed invention, the portion can be less than 1/10 the total distance 448. As such, a length of each tooth 446 can be relatively short, as compared to a length of a helical groove 452 of threads of the drive screw 406, which the tooth 446 is configured to mate (interface) with and travel (slide) along, as described further below.

As shown in FIGS. 23-25, each tooth 446 curves along the inner surface 442, to match a helical profile of the groove 452 with which it is configured to interface. For example, each tooth 446 may have a pitch and lead that matches that of the drive screw 406, as described further below. However, each tooth 446 is less than a full thread and can curve less than 90 degrees around a circumference of the proximal end 426 of the knob 404 (as shown in FIGS. 23-25). In some embodiments, each tooth 446 curves approximately or less than 45 degrees around the circumference of the proximal end 426. In some embodiments, each tooth 446 curves between 30 and 80 degrees around the circumference of the proximal end 426.

The release knob 404 is shown having two teeth 446 that are spaced apart from one another around a circumference of the inner surface 442. In some embodiments, the two teeth 446 can be spaced approximately 180 degrees apart from one another around the circumference of the inner surface 442. For example, as shown in the distal end view of FIG. 23, the two teeth 446 can include a first tooth 446a and a second tooth 446b. In alternate embodiments, such as when the drive screw is a single start screw or a different multi start screw, the release knob 404 can include only one tooth or more than two teeth (e.g., three).

In some embodiments, as shown in FIGS. 21, 22, 24, and 25, the outer surface of the main body 428 of the release knob 404 can have a curved profile (e.g. smaller diameter middle portion than the end portion of the body 428) with one or more protruding elements 450. The one or more protruding elements 450 can be configured to provide an ergonomic knob surface for a user to grip and turn. However, in alternate embodiments, the body 428 may not include the protruding elements 450 and/or may have a differently shaped profile.

The drive screw 406 can be arranged within an interior (e.g., the bore 440) of the release knob 404 (FIGS. 12-20). In some embodiments, as shown in FIGS. 26-33, the drive screw 406 can have threads defined by one or more grooves 452 indented into an outer surface 454 of a main body 456 of the drive screw 406. Each groove 452 can form a helical track, along a helical threaded portion of the main body 456 of the drive screw 406, along which a corresponding tooth 446 of the release knob 404 can travel, as the release knob 404 rotates (e.g., turns). The main body 456 further includes one or more retaining elements 458 (FIGS. 26-32). Each groove 452 can be connected to a corresponding retaining element 458 of the drive screw 406. Further, each retaining element can be arranged at a proximal end 476 of the drive screw 406 (e.g., a proximal end of the main body 456).

In some embodiments, each retaining element 458 includes a protruding member (also referred to as a detent) 460, a first linear thread portion 462 arranged on a first side of the protruding member 460 and a second linear thread portion 464 arranged on a second side of the protruding member 460 (FIGS. 26 and 27). In some embodiments, as shown in FIGS. 26, 27, and 30, the second linear thread portion 464 is connected to and continuous with a corresponding groove 452. Thus, the first linear thread portion 462 and the second linear thread portion 464 can be grooves depressed into the outer surface 454 and extending circumferentially along the main body of the drive screw, on either side of the protruding member 460. In some embodiments, the first linear thread portion 462 and the second linear thread portion 464 may not be helical (e.g., they are relatively straight or linear). As described in further detail below, when a corresponding tooth 446 of the release knob 404 is arranged in the first linear thread portion 462, the tooth 446 is trapped behind the protruding member 460, thereby maintaining the release knob 404 in a locked configuration.

In some embodiments, each retaining element 458 can include a tab 485 arranged inward (in the distal, axial direction) of the protruding member 460 (FIGS. 26, 27, 30, and 32). The tab 485 can set a length, width, and height of the cantilever of the tab 485 and protruding member 460, which sets the force required to depress the protruding member 460 and allow the tooth 446 to start traveling along the second linear thread portion 464 and groove 453. As a result, the release knob 404 can turn and cause linear travel of the drive screw 406.

Each groove 452 can extend from the second linear thread portion 464 of a corresponding retaining element 458 and helically curve around the outer surface 454 of the main body 456 of the drive screw 406, from the corresponding retaining element 458 to a distal end 474 of the main body 456.

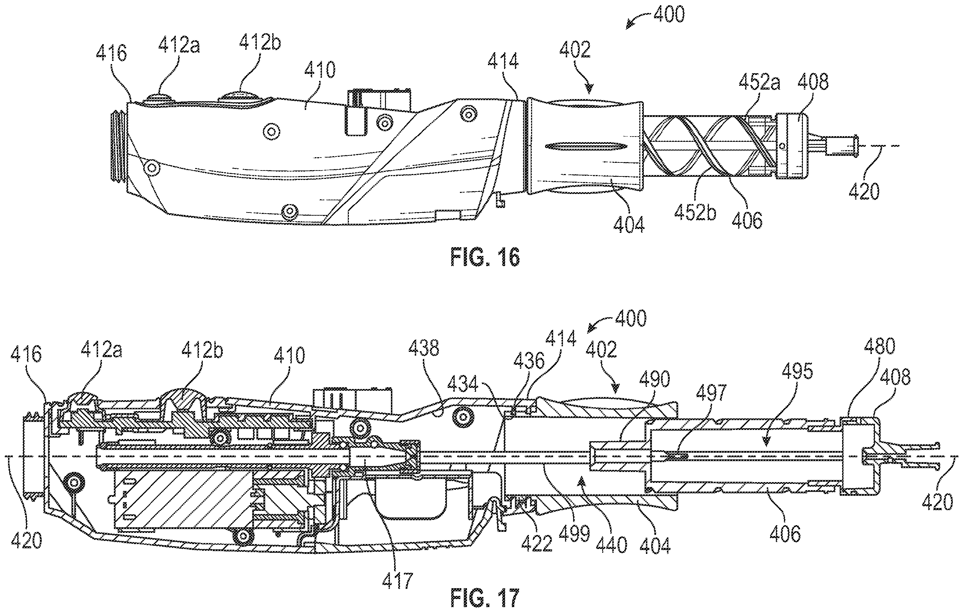

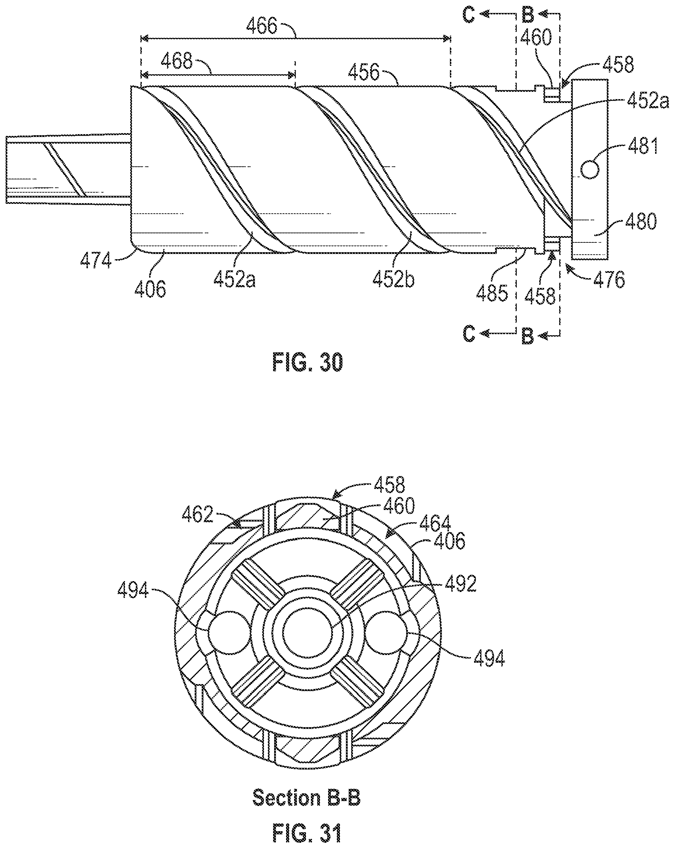

In some embodiments, as shown in FIGS. 26-33, the helical threaded portion has a double start thread formed by two helical grooves 452, including a first groove 452a and a second groove 452b (e.g., as shown in FIGS. 16 and 30). A proximal end of the first groove 452a can start at a first retaining member and a proximal end of the second groove 452b can start at a second retaining member that is spaced apart from the first retaining member. In some embodiments, as shown in FIGS. 26 and 28-33, the two retaining elements 458 can be arranged 180 degrees apart from one another around a circumference of the main body 456 of the drive screw 406.

In some embodiments, the helical threaded portion can have a lead 466 greater than 2.54cm (1 inch) and a pitch 468 greater than 1.27cm (0.5 inches). As used herein and shown in FIG. 30, the pitch 468 is the distance between a trough of one thread (groove) to the next, adjacent thread (groove) and the lead 466 is the distance along the drive screw's axis that is covered by one complete rotation of the knob (e.g., the knob tooth along the drive screw groove). In the case of a double start thread, the lead 466 is two times the pitch 468. In some embodiments, the helical threaded portion can have a lead 466 of approximately 3.81cm (1.5 inches) and a pitch 468 of approximately 1.90cm (0.75 inches). In some embodiments, the lead 466 can be in a range of 2.54-4.45cm (1-1.75 inches) and the pitch 468 can be in a range of 1.27-2.22cm (0.5-0.875 inches).

In alternate embodiments, the helical threaded portion may instead have a single start thread formed by a single groove 452. In these embodiments, the release knob 404 can include only a single tooth 446 which is configured to mate with the single groove 452. In the single start thread embodiment, the helical threaded portion can have a lead of 3.81cm (1.5 inches) and a pitch of 3.81cm (1.5 inches) or a lead of at least 2.54cm (1 inch) and a pitch of at least 2.54cm (1 inch).

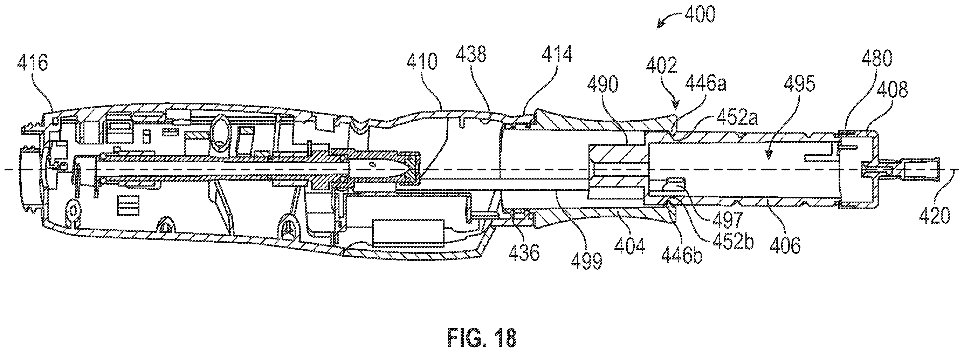

As shown in FIG. 18, each tooth 446 of the release knob 404 engages with and is configured to slide along a corresponding one of the grooves 452. For example, in the case of a double start release mechanism, as described above, a first tooth 446a of the release knob 404 engages with and slides (e.g., travels) along the first groove 452a and a second tooth 446b of the release knob 404 engages with and slides along the second groove 452b, as the release knob is turned (FIG. 18).

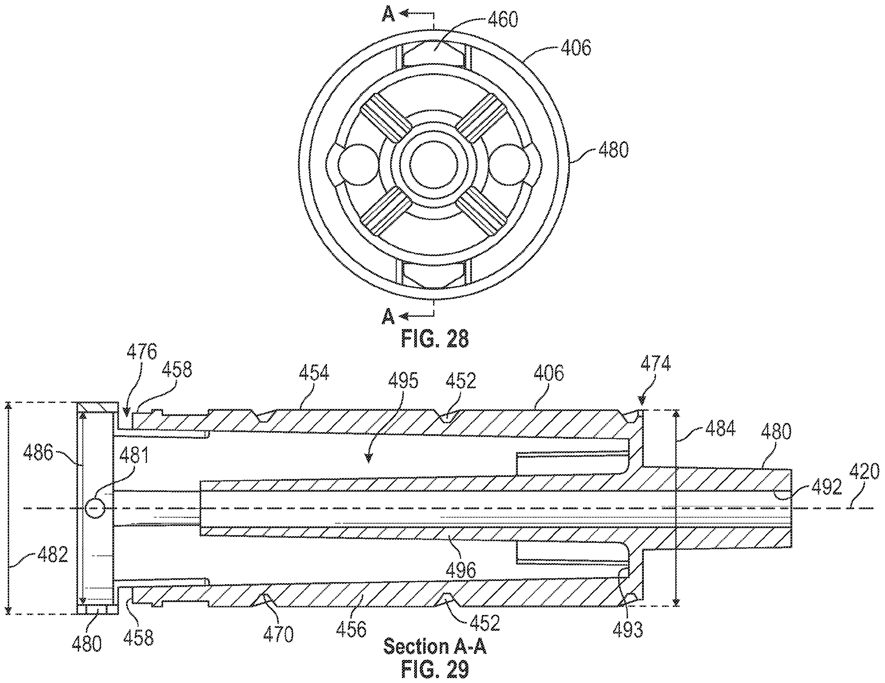

Thus, each tooth 446 can be shaped to fit within the corresponding groove 452. For example, as shown in FIG. 29, each groove 452 can have a profile 470. In some embodiments, the profile 470 can have a trapezoidal shape, such as a triangular shape with a flattened peak. As shown in FIGS. 22-25, each tooth 446 can have a profile 472 that corresponds to (e.g., matches) the profile 470 of the groove 452 such that they can fit together, while also having enough clearance therebetween to allow the tooth 446 to slide along the groove 452. For example, in some embodiments, the profile 472 can also have a trapezoidal shape, such as a triangular shape with a flattened peak. However, as explained above, each tooth 446 can be a protrusion (e.g., protrudes radially outward from the inner surface 442 of the release knob 404) while each groove 452 can be a depression (e.g., depressed into the outer surface 454 of the drive screw 406), thereby allowing each tooth 446 to extend into and mate with the corresponding groove 452.

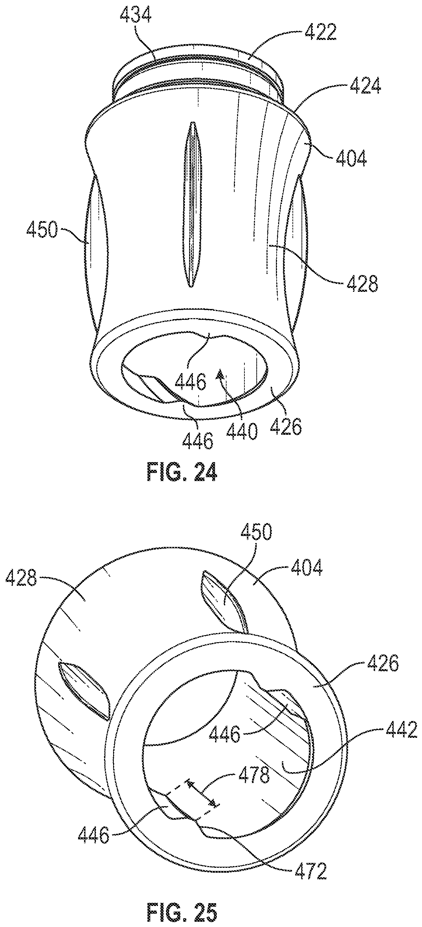

While each groove 452 curves around the outer surface 454 and extends from the proximal end 476 to the distal end 474 of the main body 456 of the drive screw 406, each tooth 446 only extends a portion of the total distance (e.g., length) 448 between the proximal end 426 and the distal end 424 of the release knob 404. Thus, a path length 478 (as shown in FIG. 25) of each tooth 446 (e.g., from its proximal end to its distal end) is relatively short compared to a path length of the corresponding groove 452.

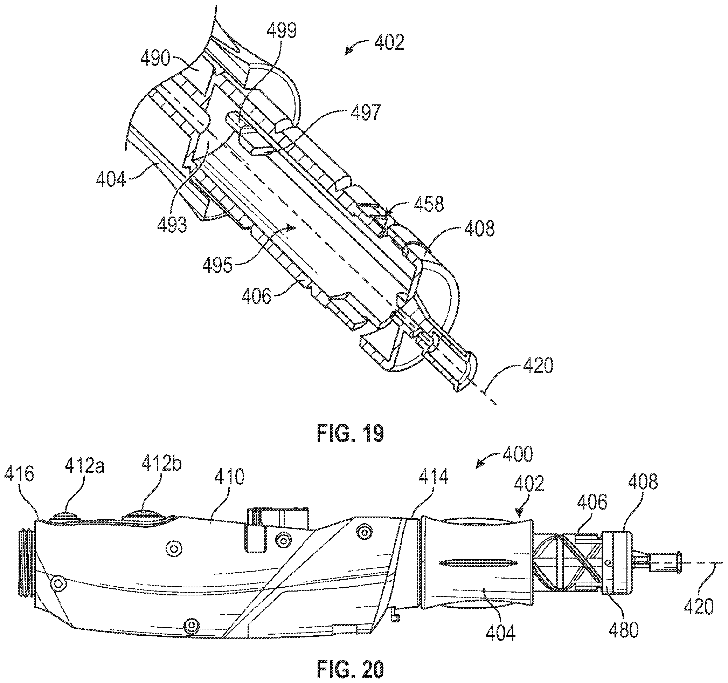

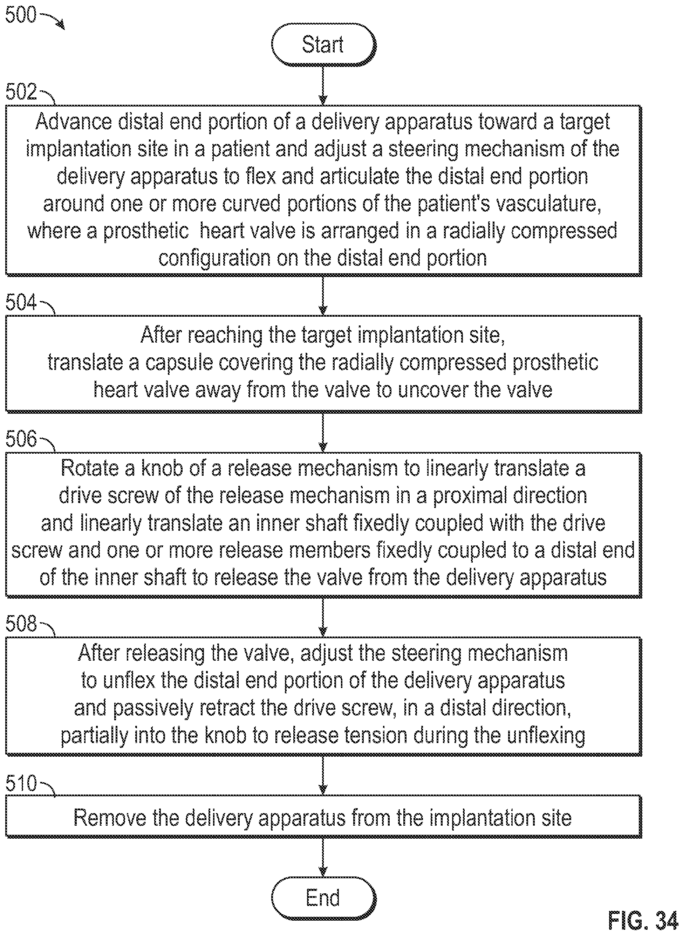

As explained further below with reference to FIG. 34, by having relatively short teeth 446 on the release knob 404 and a threaded drive screw 406 with a relatively long lead 466 and pitch 468, there may be reduced engagement between the teeth 446 of the release knob 404 and the grooves 452 of the drive screw 406 (as opposed to teeth 446 that would curve around and extend across a larger portion of the inner surface of release knob 404). This reduced level of engagement may be large enough to allow the teeth 446 to travel along the grooves 452 as the release knob is turned, until the release mechanism reaches a released configuration where the drive screw 406 extends proximally outward, in the axial direction, from the proximal end of the release knob 404 (as shown in FIGS. 16-19). At the same time, this reduced level of engagement may be small enough be allow the drive screw 406 to automatically (e.g., with manual actuation of the release knob 404) slide distally, back into the release knob 404 for tension relief in the delivery apparatus during an implantation procedure (e.g., after retracting the capsule and while unflexing the distal end portion of the delivery apparatus, as described further below, and as shown at FIG. 20).

In some embodiments, a material of the drive screw 406 and release knob 404 can also be selected to provide a desired amount of engagement between the teeth 446 of the release knob 404 and the grooves 452 of the drive screw 406. For example, in some embodiments, the materials of the drive screw 406 and at least the teeth 446 of the release knob 404 can be selected to allow the teeth 446 to slide more easily along the grooves 452. In some embodiments, the drive screw 406 and/or the release knob 404 can comprise materials that provide each of these components with relatively low friction contacting surfaces, such as thermoplastic polymers. In some embodiments, the drive screw 406 and the release knob 404 can comprise different polymeric materials (e.g., different thermoplastic polymers) that are configured to promote sliding between surfaces of the drive screw 406 and release knob 404. Possible polymeric materials can include polycarbonate, acrylonitrile butadiene styrene (ABS), polytetrafluoroethylene (PTFE), ABS impregnated with PTFE or another lubricous additive, nylon, and/or polyethylene. For example, in some embodiments, the drive screw 406 can comprise polycarbonate and the release knob can comprise ABS (or vice versa). Further, in some embodiments, together the material of the drive screw 406 and/or the release knob 404 and the lead of the grooves 452 and the teeth 446 can be selected to provide the desired level of engagement, as explained above.

As shown in FIGS. 26-33, the drive screw 406 can include a collar (or collar portion) 480 which extends proximally outward, in the axial direction, from the proximal end 476 of the main body 456. The collar 480 can be an annular collar 480 that extends around a circumference of the drive screw 406. The collar 480 can have an outer diameter 482 that is larger than an outer diameter 484 of the main body 456 (FIG. 29). The collar 480 can also have an inner surface defining an inner diameter 486 of the collar 480. The inner diameter 486 can be shaped to receive a portion of a cap 408 of the release mechanism 402 therein. For example, as shown in FIGS. 12, 13, 16-20, the cap 408 is coupled to the proximal end 476 of the main body 456 of the drive screw 406 via the collar 480. In some embodiments, the collar 480 can include one or more apertures 481 that are each configured to receive a fastener therein to couple the cap 408 to the collar 480.

In some embodiments, as shown in FIG. 13, an inner shaft 488 (e.g., inner shaft 152 of FIG. 8) can be fixedly coupled (e.g., bonded, glued, press fit, or the like) to an interior of the cap 408. For example, similar to as shown in FIGS. 8-10, the inner shaft 488 can be secured to the cap 408 and then extend through the delivery apparatus, from the cap 408 to a distal end portion or end of the delivery apparatus (e.g., to nosecone 144). The inner shaft 488 can be configured to receive a guide wire therein, and thus, in some embodiments, may be referred to as a guide wire lumen. Since the inner shaft 488 is fixedly coupled to the cap 408, which is coupled to the drive screw 406, linear translation of the drive screw 406, in the axial direction, causes linear translation of the inner shaft 488 (e.g., they translate together).

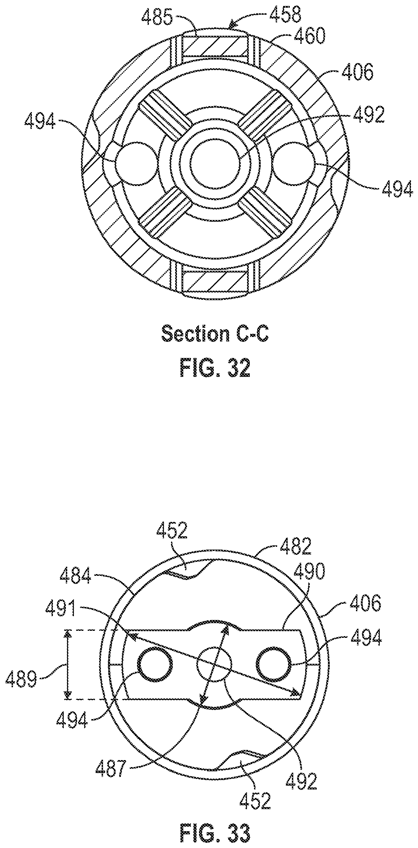

Returning to FIGS. 26-33, the drive screw 406 can further include an extension portion 490 that extend distally outward, in the axial direction, from the distal end 474 of the main body 456. The extension portion 490 can include a central bore (channel) 492 and one or more side bores 494 that are offset from the central bore 492 (as seen in the distal end view of FIG. 33). The central bore 492 can be configured to receive the inner shaft 488 therein. In some embodiments, the central bore 492 of the extension portion 490 can be continuous with and connect to a central bore portion 496 extending through an interior of the main body 456, as shown in FIG. 29. The central bore portion 496 may provide additional support to the inner shaft 488 and prevent kinking. However, in alternate embodiments, the drive screw 406 may not include the central bore portion 496.

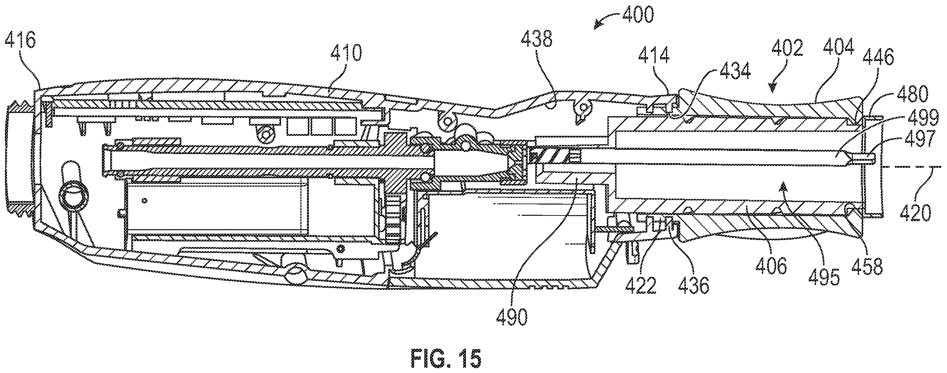

As shown in FIGS. 15 and 17-19, each of the side bores 494 can be configured to receive a corresponding rod 499 (out of two rods) therein (only one side bore 494 and rod 499 pair are shown out of two pairs). The rods 499 can be secured to an internal connecting element 417 arranged within an interior of the housing 410 of the handle portion 400 (FIG. 17).The drive screw 406 can be configured to travel linearly, in the axial direction, along the rods 499. In this way, the rods 499 may guide the linear travel of the drive screw 406 within the release knob 404.

In some embodiments, as shown in FIGS. 15 and 17-19, a proximal end of each rod 499 includes a stop (or end-of-travel) element 497 that has at least one dimension that is wider than a diameter of the rod 499. The stop element 497 can also be wider than a largest width of the internal, side bore 494 of the drive screw 406. As shown in FIGS. 15 and 17-19, the stop element 497 is arranged within an open cavity 495 of an interior of the main body 456 of the drive screw 406, the open cavity 495 arranged between the proximal end 476 and the distal end 474 of the main body 456 of the drive screw 406. The distal end 474 of the main body 456 can include an internal surface 493 that is arranged normal to the central longitudinal axis 420 (FIG. 19). Together, the internal surface 493 and the stop element 497 can create an end stop that stops the drive screw 406 from traveling any further in the proximal, axial direction. As such, the drive screw 406 can be prevented from traveling too far outside the release knob 404. In some embodiments, linear travel of the release mechanism 402 may be stopped (in or past the released configuration, as explained further herein) by the spool 212 coming into contact with the spool stop 216, at a distal end portion of the delivery apparatus (as shown in FIG. 10), prior to the stop element 497 coming into contact with the internal surface 493 (at the proximal end of the delivery apparatus).

In some embodiments, as shown in FIG. 33, the extension portion 490 can have a major axis (e.g., long dimension) with major diameter 491 and a minor axis (e.g., short dimension) with minor diameter 489. The central bore 492 and two side bores 494 can be spaced apart from one another along the major axis. The major diameter 491 can be smaller than the outer diameter 484 of the main body 456 (FIG. 33).

In some embodiments, as shown in FIG. 33, the extension portion 490 can include a bumped out (e.g., protruding) region around the central bore 492, forming a wider portion having a diameter 487 that is wider than the minor diameter 489.

FIG. 34 is a flow chart of a method 500 not covered by the present invention, for operating the handle portion 400 to deliver a prosthetic medical device (e.g., prosthetic heart valve) to a target implantation site. As described above, handle portion 400, including release mechanism 402, can be part of a delivery apparatus, such as delivery apparatus 100 of FIGS. 1-10. Method 500 may also provide a method for operating the release mechanism 402. Method 500 is described below with reference to FIGS. 9-20.

Method 500 begins at 502 and includes advancing a distal end portion of a delivery apparatus (e.g., distal end portion shown in FIGS. 9 and 10, which may be included in the delivery apparatus 100 of FIGS. 1-8) toward a target implantation site in a patient and adjust a steering mechanism of the delivery apparatus to flex and articulate the distal end portion around curved portions of the patient's vasculature. In some embodiments, an implantable medical device, such as a prosthetic heart valve (e.g., valve 10 of FIG. 1) is arranged in a radially compressed configuration on the distal end portion of the delivery apparatus. For example, the prosthetic heart valve may be contained, in its radially compressed configuration, within a capsule of the delivery apparatus (e.g., capsule 146 shown in FIGS. 2-4).

In some embodiments, the curved portion of the patient's vasculature may include an aortic arch. An example of a distal end portion of delivery apparatus being articulated around a simulated, aortic arch is shown in FIG. 11.

At 504, the method includes, after reaching the target implantation site, translating the capsule covering the radially compressed prosthetic heart valve (or alternate implantable medical device) away from the valve to uncover the prosthetic heart valve. In some embodiments, after retracting the capsule away from the radially compressed valve, the valve may self-expand to a radially expanded configuration. In some embodiments, the translating the capsule may be in response to actuation of one or more buttons on a handle portion of the delivery apparatus. For example, when a user actuates the one or more buttons, as described above with reference to FIGS. 2 and 5-7, a motor may be activated, thereby moving the capsule in the axial direction to uncover the valve.

After uncovering the prosthetic heart valve or other medical device at 504, method 500 proceeds to 506. At 506, the method includes rotating a knob of a release mechanism (e.g., release knob 404 of release mechanism 402, as shown in FIGS. 12-25) to linearly translate a drive screw (e.g., drive screw 406 shown in FIGS. 12-20 and 26-33) of the release mechanism in a proximal direction (e.g., proximal, axial direction or proximally along the axial direction) and linearly translate an inner shaft fixedly coupled with the drive screw and one or more release members fixedly coupled to a distal end of the inner shaft to release the valve from the delivery apparatus. For example, the rotating the knob to linearly translate the drive screw at 506 may include, from a starting, locked position (as shown at FIGS. 12-15), rotating the knob 404 of the valve release mechanism and moving one or more teeth 446 of the knob 404 along a corresponding groove 452 of the drive screw 406 to linearly translate the drive screw 406 proximally, in the axial direction, until the drive screw 406 reaches a released position (as shown at FIGS. 16-19).

In some embodiments, as described herein with reference to FIGS. 26-33, the drive screw 406 can have a double start thread with a relatively long lead and pitch and the knob 404 can have two teeth arranged opposite one another around a circumference of an inner surface of the knob 404 (e.g., approximately 180 degrees apart). The thread of the drive screw 406 can be defined by two helical grooves 452. Each tooth 446 is configured to mate with and translate (e.g., travel or slide) along a corresponding groove 452.

As shown in FIGS. 12-15, in the starting, locked position or configuration, the drive screw 406 is retracted within the handle portion 400 and a majority of the main body 456 of the drive screw 406 is arranged within an interior of the knob 404. For example, only the collar 480 of the drive screw 406 may extend axially outside, in the proximal direction, of the knob 404 in the starting, locking position.

Further, in the starting, locked position or configuration, each tooth 446 can be arranged within a first linear thread portion 462 of a corresponding retaining element 458 (as shown at FIG. 15). Each tooth 446 may be retained in the retaining element 458 via a protruding member 460 of the retaining element 458 which protrudes radially outward relative to the first linear thread portion 462. The protruding member 460 prevents rotation of the knob 404 in response to forces generated by articulating (e.g., flexing) the distal end portion of the delivery apparatus during the method at 502. In this way, accidental, premature release of the prosthetic heart valve from the delivery apparatus (via unintended actuation of the knob 404) may be prevented.

Upon initial rotation of the knob 404 (e.g., via a user), each tooth 446 can overcome the protruding member 460 of the retaining element 458, by moving over and past the protruding member 460, to the second linear thread portion 464 which is connected to a corresponding groove 452. In this way, rotating the knob at 506 may first include initially rotating the knob to uncouple (or release) the teeth 446 from the corresponding retaining element 458. A user may feel an initial resistance in overcoming the retaining element 458, due to the protruding member 460. However, after passing over the protruding member 460, the user may feel less resistance in turning the knob 404. The tooth 446 can then slide and travel along the path of the groove, as the knob 404 is turned. As the tooth 446 travels along the groove 452, in response to the knob 404 turning, the drive screw 406 translates proximally in the axial direction while the axial position of the knob 404 remains fixed. For example, the proximal end 476 of the drive screw 406 extends further outside of the knob 404, as each tooth 446 continues to travel along the corresponding groove 452.

The method at 506 can further include, while the drive screw 406 is translating in the proximal, axial direction, linearly translating the inner shaft 488 fixedly coupled with the drive screw 406 (FIG. 13) and one or more release members fixedly coupled to a distal end of the inner shaft (e.g., release members 208 coupled to shaft 152 in FIGS. 9 and 10) to release a valve from the delivery apparatus. For example, as explained above with reference to FIG. 13, a proximal end of the inner shaft 488 may be fixedly coupled to the cap 408 which is coupled to the proximal end of the drive screw 406 and release members, such as release members 208, may be fixedly coupled to a distal end portion of the inner shaft 488. Thus, linear translation of the drive screw 406 in the proximal, axial direction results in linear translation of the release members in the proximal, axial direction (e.g., these components move together). As explained above, by moving the release members in the proximal, axial direction, away from the prosthetic heart valve (or other implantable medical device), the release members become uncoupled from the prosthetic heart valve, thereby disconnecting and releasing the prosthetic heart valve from the delivery apparatus.

In the released position or configuration of the release mechanism 402 (and the drive screw 406), the drive screw 406 extends proximally outward, in the axial direction, from the proximal end of the release knob 404 (as shown in FIGS. 16-19). Further, the teeth 446 of the release knob 404 may be engaged with corresponding grooves 452 of the drive screw 406, at or proximate to a distal end 474 of the main body 456 of the drive screw 406 (as shown in FIG. 18). In this released position, the stop element 497 of the rod 499 may be arranged close to the internal surface 493 of the distal end 474 of the main body 456 (as shown in FIGS. 18 and 19). Also, in this released configuration, the release members (e.g., release members 208) are arranged away from the prosthetic heart valve. For example, as shown in FIG. 10, in the released configuration, the spool 212 may be arranged proximate to or in contact with the spool stop 216, and thus, the release members 208 may be arranged closer to the spool stop 216 (as compared to the starting configuration shown in FIG. 9).

Continuing to 508, the method can include, after releasing the prosthetic heart valve (or other implantable medical device), adjusting (e.g., actuating) the steering mechanism to unflex the distal end portion of the delivery apparatus and passively retracting the drive screw in a distal, axial direction, partially into the knob to release tension during the unflexing. As explained above, tension in the shafts of the delivery apparatus can be created during releasing the valve from the delivery apparatus, while the distal end portion of the delivery apparatus is being unflexed. Thus, this tension needs to be relieved to enable complete and successful unflexing of the distal end portion of the delivery apparatus and removal of the delivery apparatus from the implantation site. By allowing the drive screw 406 to passively retract into the knob 404, thereby linearly translating the inner shaft 488 distally, in the axial direction, the tension created during the unflexing can be relieved, as the distal end portion of the delivery apparatus is unflexed (e.g., via actuating the steering mechanism, such as via rotation of the steering knob 418).

For example, from the released configuration of the release mechanism 402 (shown at FIGS. 16-19), as the distal end portion of the delivery apparatus is unflexed (e.g., as the steering knob 418 is rotated), the drive screw can translate in distal, axial direction, at least partially back into the interior of the release knob 404, as shown at FIG. 20. As used herein, "passively" refers to free movement of the drive screw, without manual actuation of the knob 404 (e.g., via a user turning the knob 404). During the unflexing the distal end portion of the delivery apparatus, the pulling forces at the distal end portion of the delivery apparatus may be large enough to overcome the resistance of the mating connection between the teeth 446 of the knob 404 and the grooves 452 of the drive screw 406, thereby allowing the drive screw 406 to be pulled back into the knob 404. As the drive screw 406 is pulled back into the knob 404, the teeth 446 move along the grooves 452, which passively turns the knob 404. In this way, tension accumulated at the distal end portion of the delivery apparatus may be automatically relieved via the release mechanism 402 (without user intervention), and the distal end portion of the delivery apparatus may be unflexed and removed from the implantation site and the patient. This may simplify the implantation process, while allowing a user to more easily remove the delivery apparatus from the implantation site. As introduced above, the relatively short teeth 446 of the knob 404 and the long lead/pitch of the grooves 452 of the drive screw 406, along with lower friction contact surfaces of the knob 404 and drive screw 406, enable the drive screw 406 to be passively pulled back into the knob to relieve tension during the unflexing.

Thus, the method at 510 includes, after unflexing the distal end portion of the delivery apparatus at 508, removing the delivery apparatus from the implantation site (and the patient).

In view of the many possible embodiments to which the principles of the disclosed technology may be applied, it should be recognized that the illustrated embodiments are only preferred examples of the disclosed technology and should not be taken as limiting the scope of the claimed subject matter. Rather, the scope of the claimed subject matter is defined by the following claims.

## Claims

1. A delivery apparatus (100) for an expandable, implantable medical device (10), the delivery apparatus comprising:
a handle portion (132, 400) including a release mechanism (200, 402) configured to adjust a linear position, relative to a central longitudinal axis (L-L') of the delivery apparatus (100), of a component of the delivery apparatus (100), the release mechanism (200, 402) comprising:
a threaded drive screw (161, 406) including a helical threaded portion having a lead (466) of at least 2.54 cm (one inch), where the helical threaded portion includes one or more grooves (452) extending around the drive screw (161, 406), the drive screw (161, 406) coupled to the component; and
a rotatable knob (136, 404) surrounding and coaxial with the drive screw (161, 406), the knob (136, 404) including one or more teeth (446) arranged at a proximal end of the knob (136, 404), each tooth (446)of the one or more teeth (446) configured to interface with a corresponding groove of the one or more grooves (452) of the drive screw (161, 406), wherein each tooth (446) of the one or more teeth (446) extends from the proximal end, toward a distal end (424) of the knob (136, 404), only a portion of a total distance between the proximal end (426) and the distal end (424), wherein the portion is less than ¼ the total distance (448).

2. The delivery apparatus (100) of claim 1, wherein the threaded drive screw (161, 406) includes one or more retaining elements (458) arranged at a proximal end (476) of the drive screw (161, 406), wherein the one or more grooves (452) of the helical threaded portion are each connected to a corresponding retaining element (458) of the one or more retaining elements (458) and extend around the drive screw (161, 406), from the corresponding retaining element (458) to a distal end (474) of the drive screw (161, 406), and wherein each tooth (446) of the one or more teeth (446) is configured to interface with a corresponding retaining element (458).

3. The delivery apparatus (100) of claim 2, wherein each tooth (446) is configured to mate with and travel along the corresponding groove (452), as the knob (136, 404) is rotated about the central longitudinal axis (L-L'), wherein the drive screw (161, 406) is configured to move linearly, in an axial direction relative to the knob (136, 404), as the knob (136, 404) rotates and the tooth (446) travels along the corresponding groove (452), wherein the knob (136, 404) is fixed from translating in the axial direction, wherein the axial direction is relative to the central longitudinal axis (L-L'), wherein the knob (136, 404) includes a collar (422) extending distally from the distal end (424) of the knob (136, 404) into an interior of a housing (410) of the handle portion (132, 400), the collar (422) including one or more collar grooves (434) extending around a circumference of the collar (422), each collar groove (434) mating with a corresponding annular protrusion (436) extending radially from an inner surface of the housing (410) of the handle portion (132, 400), and wherein the knob (136, 404) is fixed from translating in the axial direction and is configured to rotate about the central longitudinal axis (L-L'), relative to the housing (410) of the handle portion (132, 400), via a mating connection between each collar groove (434) and the corresponding annular protrusion (436).

4. The delivery apparatus (100) of either claim 2 or claim 3, wherein the drive screw (161, 406) is linearly movable between a starting, locked configuration where each tooth (446) is coupled to the corresponding retaining element (458) and an entirety of the helical threaded portion of the drive screw (161, 406) is arranged within an interior of the knob (136, 404) and the handle portion (132, 400) and a released configuration where each tooth (446) is mated with a distal portion of the corresponding groove (452), the distal portion arranged closer to the distal end (474) than the proximal end (476) of the drive screw (161, 406), and a majority of the helical threaded portion of the drive screw (161, 406) extends outward of the proximal end (426) of the knob (136, 404), in the axial direction.

5. The delivery apparatus (100) of claim 4, wherein each retaining element (458) includes a protruding member (460), a first linear thread portion (462) arranged on a first side of the protruding member (460), and a second linear thread portion (464) arranged on a second side of the protruding member (460), the second linear thread portion (464) connected to and continuous with a corresponding groove (452) of the one or more grooves (452) of the drive screw (161, 406) and wherein in the starting, locked configuration, each tooth is arranged within the first linear thread portion (462) of the corresponding retaining element (458).

6. The delivery apparatus (100) of any one of claims 2-5, further comprising a rod (499) including a distal end fixedly coupled to an internal surface of the handle portion (132, 400) and wherein the drive screw (161, 406) includes an extension portion (490) extending axially outward from the distal end (474) of the drive screw (161, 406), the extension portion (490) including an internal bore (494) mounted around the rod (499) and configured to slide linearly along the rod (499),
optionally wherein a proximal end of the rod (499) includes a stop element (497) that is wider than a largest width of the internal bore of the drive screw (161, 406) and wherein the stop element (497) is arranged within an open cavity (495) of an interior of the drive screw (161, 406), the open cavity (495) arranged between the proximal end (476) and the distal end (474) of the drive screw (161, 406).

7. The delivery apparatus (100) of claim 6, wherein the extension portion (490) further includes a central bore (492) centered along the central longitudinal axis (L-L'), where the internal bore is radially offset from the central bore (492), and wherein the central bore (492) is configured to receive an inner shaft (152) of the delivery apparatus (100) therethrough.

8. The delivery apparatus (100) of any one of claims 1-7, wherein the component of the delivery apparatus (100) which the release mechanism (200, 402) is configured to adjust the linear position of includes one or more release members (156) removably coupled to the implantable medical device (10),
optionally further comprising an inner shaft (152, 488) including a proximal end fixedly coupled to a cap (408) of the release mechanism (200, 402), the cap (408) coupled to the proximal end (476) of the drive screw (161, 406), wherein the inner shaft extends to a distal end of the delivery apparatus (100), and wherein the one or more release members (156) are fixedly coupled to a distal end portion of the inner shaft (152, 488).

9. The delivery apparatus (100) of any one of claims 1-8, wherein threads formed by the one or more grooves (452) of the helical threaded portion are double start threads formed by two grooves (452) and wherein the drive screw (161, 406) has two retaining elements (458), each groove (452) extending from a different one of the two retaining elements (458), and wherein the knob (136, 404) includes two teeth (446), each configured to mate with and slide along a different one of the two grooves (452), the two teeth (446) spaced apart from one another around a circumference of the proximal end (426) of the knob (136, 404).

10. The delivery apparatus (100) of any one of claims 1-9, wherein the handle portion (132, 400) further comprises a steering mechanism including a steering knob (418) configured to rotate relative to a housing (410) of the handle portion (132, 400) and adjust a curvature of one or more shafts (134, 150, 152) of the delivery apparatus (100), at a distal end portion of the delivery apparatus (100).

11. The delivery apparatus (100) of any one of claims 1-10, wherein the implantable medical device (10) is a prosthetic heart valve configured to radially self-expand to a functional size of the prosthetic heart valve.

12. The delivery apparatus (100) of any one of claims 1-11, wherein the portion of the total distance (448) between the proximal end (426) and the distal end (424) is less than 1/10 the total distance (448).

13. The delivery apparatus (100) of claim 1, the delivery apparatus (100) further comprising:
an inner shaft (152, 488); and
one or more release members (156, 208), each including a proximal end coupled to an outer surface of a distal end portion of the inner shaft (152, 488) and a distal end configured to be releasably coupled to the implantable medical device (10) arranged around the distal end portion of the inner shaft (152, 488), distal to where the proximal end couples to the inner shaft (152, 488);
wherein the handle portion (132, 400) further includes:
a steering mechanism configured to adjust a curvature of and flex one or more shafts (134, 150, 152) of the delivery apparatus (100), including the inner shaft (152, 488), at a distal end portion of the delivery apparatus (100);
wherein the release mechanism (200, 402) is configured to adjust a linear position of the inner shaft (152, 488) and the one or more release members (156), along a central longitudinal axis (L-L') of the delivery apparatus (100), relative to an outer housing (410) of the handle portion (132, 400);
wherein the threaded drive screw (161, 406) is coupled to a proximal end of the inner shaft (152, 488) and the helical threaded portion is arranged in a main body (456) of the drive screw (161, 406), wherein the one or more grooves (452) forming the helical threaded portion extend from a proximal end (476) to a distal end (474) of the main body (456) of the drive screw (161, 406); and
wherein the rotatable knob (136, 404) comprises a rotatable release knob (136, 404) coupled to the outer housing (410) of the handle portion (132, 400) and wherein the drive screw (161, 406) is adapted to move linearly, along the central longitudinal axis (L-L'), relative to the release knob (136, 404), in response to rotation of the release knob (136, 404) and sliding of the one or more teeth (446) along the one or more grooves (452), and wherein actuating the steering mechanism to unflex the one or more shafts (134, 150, 152) of the delivery apparatus (100) allows the drive screw (161, 406) to move distally, along the central longitudinal axis (L-L'), to release tension created in the distal end portion of the delivery apparatus (100),
optionally wherein each tooth (446) of the one or more teeth (446) of the release knob (136, 404) is less than a full thread and curves less than 45 degrees around a circumference of the proximal end (426) of the release knob (136, 404).

14. The delivery apparatus (100) of claim 13, wherein the main body (456) of the drive screw (161, 406) further includes one or more retaining elements (458) arranged at the proximal end (476) of the main body (456), where each groove (452) of the one or more grooves (452) of the helical threaded portion is connected to a corresponding retaining element (458) of the one or more retaining elements (458) and curves around an outer surface of the main body (456) and extends from the retaining element (458) to the distal end (474) of the main body (456),
optionally wherein the drive screw (161, 406) is linearly movable between a starting, locked configuration where each tooth (446) of the one or more teeth (446) is coupled to a corresponding retaining element (458) of the one or more retaining elements (458) and an entirety of the helical threaded portion of the drive screw (161, 406) is arranged within an interior of the release knob (136, 404) and the handle portion (132, 400) and a released configuration where each tooth (446) is mated with a distal portion of a corresponding groove (452) of the one or more grooves (452), the distal portion arranged closer to the distal end than the proximal end (426) of the main body (428), and a majority of the helical threaded portion of the drive screw (161, 406) extends outward of the proximal end (426) of the release knob (136, 404), in an axial direction.

15. The delivery apparatus (100) of claim 13 or claim 14, wherein threads formed by the one or more grooves (452) of the helical threaded portion are double start threads formed by two grooves (452), wherein the release knob (136, 404) includes two teeth (446), each configured to mate with and slide along a different one of the two grooves (452), wherein the two teeth (446) are spaced apart from one another around a circumference of the proximal end (426) of the release knob (136, 404), and wherein the threads of the drive screw (161, 406) have a lead in a range of 2,54 to 4.45 cm (1 to 1.75 inches).

## Patentansprüche

1. Zuführeinrichtung (100) für eine expandierbare implantierbare medizinische Vorrichtung (10), wobei die Zuführeinrichtung umfasst:
einen Griffabschnitt (132, 400), der einen Freigabemechanismus (200, 402) einschließt, der ausgestaltet ist, um eine lineare Position relativ zu einer zentralen Längsachse (L-L') der Zuführeinrichtung (100) von einer Komponente der Zuführeinrichtung (100) anzupassen, wobei der Freigabemechanismus (200, 402) umfasst:
eine Gewindeantriebsspindel (161, 406), die einen helixförmigen Gewindeabschnitt mit einer Steigung (466) von mindestens 2,54 cm (einem Zoll) einschließt, wobei der helixförmige Gewindeabschnitt eine oder mehrere Rillen (452) einschließt, die sich um die Antriebsspindel (161, 406) erstrecken, wobei die Antriebsspindel (161, 406) an die Komponente gekoppelt ist; und
einen rotierbaren Knopf (136, 404), der die Antriebsspindel (161, 406) umgibt und koaxial damit ist, wobei der Knopf (136, 404) einen Zahn oder mehrere Zähne (446) einschließt, der/die an einem proximalen Ende des Knopfes (136, 404) angeordnet ist/sind, wobei jeder Zahn (446) des einen Zahns oder der mehrere Zähne (446) ausgestaltet ist, um mit einer entsprechenden Rille der einen oder mehreren Rillen (452) der Antriebsspindel (161, 406) zu koppeln, wobei jeder Zahn (446) des einen Zahns oder der mehreren Zähne (446) sich von dem proximalen Ende in Richtung eines distalen Endes (424) des Knopfes (136, 404) über nur einen Abschnitt eines Gesamtabstands zwischen dem proximalen Ende (426) und dem distalen Ende (424) erstreckt, wobei der Abschnitt kleiner als 1/4 des Gesamtabstands (448) ist.

2. Zuführeinrichtung (100) nach Anspruch 1, wobei die Gewindeantriebsspindel (161, 406) ein oder mehrere Halteelemente (458) einschließt, das/die an einem proximalen Ende (476) der Antriebsspindel (161, 406) angeordnet ist/sind, wobei die eine oder mehreren Rillen (452) des helixförmigen Gewindeabschnitts jeweils mit einem entsprechenden Halteelement (458) des einen Halteelements oder der mehreren Halteelemente (458) verbunden ist/sind und sich um die Antriebsspindel (161, 406) herum von dem entsprechenden Halteelement (458) zu einem distalen Ende (474) der Antriebsspindel (161, 406) erstreckt/erstrecken, und wobei jeder Zahn (446) des einen Zahns oder der mehreren Zähne (446) ausgestaltet ist, um mit einem entsprechenden Halteelement (458) zu koppeln.

3. Zuführeinrichtung (100) nach Anspruch 2, wobei jeder Zahn (446) ausgestaltet ist, um mit der entsprechenden Rille (452) gepaart zu werden und sich entlang dieser zu bewegen, wenn der Knopf (136, 404) um die zentrale Längsachse (L-L') rotiert wird, wobei die Antriebsspindel (161, 406) ausgestaltet ist, um sich linear in eine axiale Richtung relativ zu dem Knopf (136, 404) zu bewegen, wenn der Knopf (136, 404) rotiert und der Zahn (446) sich an der entsprechenden Rille (452) entlang bewegt, wobei der Knopf (136, 404) so fixiert ist, dass er nicht in die axiale Richtung translatiert, wobei die axiale Richtung relativ zu der zentralen Längsachse (L-L') ist, wobei der Knopf (136, 404) einen Kragen (422) einschließt, der sich distal von dem distalen Ende (424) des Knopfes (136, 404) zu einem Inneren eines Gehäuses (410) des Griffabschnitts (132, 400) erstreckt, wobei der Kragen (422) eine oder mehrere Kragenrillen (434) einschließt, die sich um einen Umfang des Kragens (422) erstrecken, wobei jede Kragenrille (434) mit einem entsprechenden kranzförmigen Vorsprung (436) gepaart wird, der sich radial von einer Innenoberfläche des Gehäuses (410) des Griffabschnitts (132, 400) erstreckt, und wobei der Knopf (136, 404) so fixiert ist, dass er nicht in die axiale Richtung translatiert, und so ausgestaltet ist, dass er um die zentrale Längsachse (L-L') relativ zu dem Gehäuse (410) des Griffabschnitts (132, 400) mittels einer paarigen Verbindung zwischen jeder Kragenrille (434) und dem entsprechenden kranzförmigen Vorsprung (436) rotiert.

4. Zuführeinrichtung (100) nach Anspruch 2 oder Anspruch 3, wobei die Antriebsspindel (161, 406) linear zwischen einer verriegelten Ausgangskonfiguration, in der jeder Zahn (446) an das entsprechende Halteelement (458) gekoppelt ist, und eine Gesamtheit des helixförmigen Gewindeabschnitts der Antriebsspindel (161, 406) innerhalb eines Inneren des Knopfes (136, 404) und des Griffabschnitts (132, 400) angeordnet ist, und einer freigegebenen Konfiguration bewegbar ist, in der jeder Zahn (446) mit einem distalen Abschnitt der entsprechenden Rille (452) gepaart ist, wobei der distale Abschnitt näher an dem distalen Ende (474) als dem proximalen Ende (476) der Antriebsspindel (161, 406) angeordnet ist, und ein überwiegender Teil des helixförmigen Gewindeabschnitts der Antriebsspindel (161, 406) sich auswärts von dem proximalen Ende (426) des Knopfes (136, 404) in die axiale Richtung erstreckt.

5. Zuführeinrichtung (100) nach Anspruch 4, wobei jedes Halteelement (458) ein vorspringendes Element (460), einen ersten linearen Gewindeabschnitt (462), der auf einer ersten Seite des vorspringenden Elements (460) angeordnet ist, und einen zweiten linearen Gewindeabschnitt (464) einschließt, der auf einer zweiten Seite des vorspringenden Elements (460) angeordnet ist, wobei der zweite lineare Gewindeabschnitt (464) mit einer entsprechenden Rille (452) der einen oder mehreren Rillen (452) der Antriebsspindel (161, 406) verbunden und kontinuierlich damit ist, und wobei in der verriegelten Ausgangskonfiguration jeder Zahn innerhalb des ersten linearen Gewindeabschnitts (462) des entsprechenden Halteelements (458) angeordnet ist.

6. Zuführeinrichtung (100) nach einem der Ansprüche 2 bis 5, des Weiteren umfassend einen Stab (499), der ein distales Ende einschließt, das fest an eine Innenoberfläche des Griffabschnitts (132, 400) gekoppelt ist, und wobei die Antriebsspindel (161, 406) einen Fortsatzabschnitt (490) einschließt, der sich axial auswärts von dem distalen Ende (474) der Antriebsspindel (161, 406) erstreckt, wobei der Fortsatzabschnitt (490) eine Innenbohrung (494) einschließt, die um den Stab (499) montiert ist und ausgestaltet ist, um linear an dem Stab (499) entlang zu gleiten,
wobei gegebenenfalls ein proximales Ende des Stabs (499) ein Anschlagelement (497) einschließt, das breiter als eine größte Breite der Innenbohrung der Antriebsspindel (161, 406) ist, und wobei das Anschlagelement (497) innerhalb eines offenen Hohlraums (495) eines Inneren der Antriebsspindel (161, 406) angeordnet ist, wobei der offene Hohlraum (495) zwischen dem proximalen Ende (476) und dem distalen Ende (474) der Antriebsspindel (161, 406) angeordnet ist.

7. Zuführeinrichtung (100) nach Anspruch 6, wobei der Fortsatzabschnitt (490) des Weiteren eine zentrale Bohrung (492) einschließt, die entlang der zentralen Längsachse (L-L') zentriert ist, wobei die Innenbohrung radial von der zentralen Bohrung (492) versetzt ist, und wobei die zentrale Bohrung (492) ausgestaltet ist, um einen Innenschaft (152) der Zuführeinrichtung (100) dort hindurch aufzunehmen.

8. Zuführeinrichtung (100) nach einem der Ansprüche 1 bis 7, wobei die Komponente der Zuführeinrichtung (100), deren lineare Position der Freigabemechanismus (200, 402) konfigurationsgemäß anpassen soll, ein oder mehrere Freigabeelemente (156) einschließt, die entfernbar an die implantierbare medizinische Vorrichtung (10) gekoppelt sind,
gegebenenfalls des Weiteren umfassend einen Innenschaft (152, 488), der ein proximales Ende einschließt, das fest an eine Kappe (408) des Freigabemechanismus (200, 402) gekoppelt ist, wobei die Kappe (408) an das proximale Ende (476) der Antriebsspindel (161, 406) gekoppelt ist, wobei der Innenschaft sich zu einem distalen Ende der Zuführeinrichtung (100) erstreckt, und wobei das eine oder die mehreren Freigabeelemente (156) fest an einen distalen Endabschnitt des Innenschafts (152, 488) gekoppelt ist/sind.

9. Zuführeinrichtung (100) nach einem der Ansprüche 1 bis 8, wobei das durch die eine oder mehreren Rillen (452) des helixförmigen Gewindeabschnitt gebildete Gewinde ein Doppelganggewinde ist, das durch zwei Rillen (452) gebildet ist, und wobei die Antriebsspindel (161, 406) zwei Halteelemente (458) auf-weist, wobei jede Rille (452) sich von einem anderen der zwei Halteelemente (458) erstreckt, und wobei der Knopf (136, 404) zwei Zähne (446) einschließt, von denen jeder ausgestaltet ist, um mit einer anderen der zwei Rillen (452) zu paaren und entlang dieser zu gleiten, wobei die zwei Zähne (446) voneinander um einen Umfang des proximalen Endes (426) von dem Knopf (136, 404) beabstandet sind.

10. Zuführeinrichtung (100) nach einem der Ansprüche 1 bis 9, wobei der Griffabschnitt (132, 400) des Weiteren einen Lenkmechanismus umfasst, der einen Lenkknopf (418) einschließt, der ausgestaltet ist, um relativ zu einem Gehäuse (410) des Griffabschnitts (132, 400) zu rotieren und eine Krümmung des einen Schafts oder der mehreren Schäfte (134, 150, 152) der Zuführeinrichtung (100) an einem distalen Endabschnitt der Zuführeinrichtung (100) anzupassen.

11. Zuführeinrichtung (100) nach einem der Ansprüche 1 bis 10, wobei die implantierbare medizinische Vorrichtung (10) eine prothetische Herzklappe ist, die ausgestaltet ist, um sich radial selbst auf eine funktionelle Größe der prothetischen Herzklappe zu expandieren.

12. Zuführeinrichtung (100) nach einem der Ansprüche 1 bis 11, wobei der Abschnitt des Gesamtabstands (448) zwischen dem proximalen Ende (426) und dem distalen Ende (424) weniger als 1/10 des Gesamtabstands (448) beträgt.

13. Zuführeinrichtung (100) nach Anspruch 1, wobei die Zuführeinrichtung (100) des Weiteren umfasst:
einen Innenschaft (152, 488); und
ein oder mehrere Freigabeelemente (156, 208), das/die jeweils ein proximales Ende, das an eine Außenoberfläche eines distalen Endabschnitts des Innenschafts (152, 488) gekoppelt ist, und ein distales Ende einschließt/einschließen, das ausgestaltet ist, um freigebbar an die implantierbare medizinische Vorrichtung (10) gekoppelt zu werden, die um den distalen Endabschnitt des Innenschafts (152, 488) distal zu der Stelle angeordnet ist, an der das proximale Ende an den Innenschaft (152, 488) koppelt;
wobei der Griffabschnitt (132, 400) des Weiteren einschließt:
einen Lenkmechanismus, der ausgestaltet ist, um eine Krümmung von einem Schaft oder mehreren Schäften (134, 150, 152) der Zuführeinrichtung (100) einschließlich des Innenschafts (152, 488) an einem distalen Endabschnitt der Zuführeinrichtung (100) anzupassen und diesen/diese zu biegen;
wobei der Freigabemechanismus (200, 402) ausgestaltet ist, um eine lineare Position des Innenschafts (152, 488) und des einen Freigabeelements oder der mehreren Freigabeelemente (156) entlang einer zentralen Längsachse (L-L') der Zuführeinrichtung (100) relativ zu einem Außengehäuse (410) des Griffabschnitts (132, 400) anzupassen;
wobei die Gewindeantriebsspindel (161, 406) an ein proximales Ende des Innenschafts (152, 488) gekoppelt ist, und der helixförmige Gewindeabschnitt in einem Hauptkörper (456) der Antriebsspindel (161, 406) angeordnet ist, wobei die eine oder mehreren Rillen (452), die den helixförmigen Gewindeabschnitt bilden, sich von einem proximalen Ende (476) zu einem distalen Ende (474) des Hauptkörpers (456) der Antriebsspindel (161, 406) erstreckt/erstrecken; und
wobei der rotierbare Knopf (136, 404) einen rotierbaren Freigabeknopf (136, 404) umfasst, der an das Außengehäuse (410) des Griffabschnitts (132, 400) gekoppelt ist, und wobei die Antriebsspindel (161, 406) angepasst ist, um sich entlang der zentralen Längsachse (L-L') relativ zu dem Freigabeknopf (136, 404) in Reaktion auf Rotation des Freigabeknopfes (136, 404) und Gleiten des einen Zahns oder der mehreren Zähne (446) entlang der einen oder mehreren Rillen (452) linear zu bewegen, und wobei Betätigen des Lenkmechanismus, um die Biegung aus dem einen Schaft oder den mehreren Schäften (134, 150, 152) der Zuführeinrichtung (100) heraus zu nehmen, gestattet, dass sich die Antriebsspindel (161, 406) distal entlang der zentralen Längsachse (L-L') bewegt, um mechanische Spannung zu lösen, die in dem distalen Endabschnitt der Zuführeinrichtung (100) erzeugt wurde,
wobei gegebenenfalls jeder Zahn (446) des einen Zahns oder der mehreren Zähne (446) des Freigabeknopfes (136, 404) weniger als ein Vollgewinde ist und sich um weniger als 45 Grad um einen Umfang des proximalen Endes (426) des Freigabeknopfes (136, 404) krümmt.

14. Zuführeinrichtung (100) nach Anspruch 13, wobei der Hauptkörper (456) der Antriebsspindel (161, 406) des Weiteren ein oder mehrere Halteelemente (458) einschließt, das/die an dem proximalen Ende (476) des Hauptkörpers (456) angeordnet ist/sind, wobei jede Rille (452) der einen oder mehreren Rillen (452) des helixförmigen Gewindeabschnitts mit einem entsprechenden Halteelement (458) des einen oder der mehreren Halteelemente (458) verbunden ist und sich um eine Außenoberfläche des Hauptkörpers (456) krümmt und von dem Halteelement (458) zu dem distalen Ende (474) des Hauptkörpers (456) erstreckt,
wobei gegebenenfalls die Antriebsspindel (161, 406) linear zwischen einer verriegelten Ausgangskonfiguration, in der jeder Zahn (446) des einen Zahns oder der mehreren Zähne (446) an ein entsprechendes Halteelement (458) des einen oder mehrerer Halteelemente (458) gekoppelt ist, und eine Gesamtheit des helixförmigen Gewindeabschnitts der Antriebsspindel (161, 406) innerhalb eines Inneren des Freigabeknopfes (136, 404) und des Griffabschnitts (132, 400) angeordnet ist, und einer freigegebenen Konfiguration bewegbar ist, in der jeder Zahn (446) mit einem distalen Abschnitt der entsprechenden Rille (452) der einen oder mehreren Rillen (452) gepaart ist, wobei der distale Abschnitt näher an dem distalen Ende als dem proximalen Ende (426) des Hauptkörpers (428) angeordnet ist, und ein überwiegender Teil des helixförmigen Gewindeabschnitts der Antriebsspindel (161, 406) sich auswärts von dem proximalen Ende (426) des Knopfes (136, 404) in die axiale Richtung erstreckt.

15. Zuführeinrichtung (100) nach Anspruch 13 oder Anspruch 14, wobei das aus der einen oder den mehreren Rillen (452) des helixförmigen Gewindeabschnitts gebildete Gewinde ein Doppelganggewinde ist, das durch zwei Rillen (452) gebildet ist, wobei der Freigabeknopf (136, 404) zwei Zähne (446) einschließt, die jeweils ausgestaltet sind, um mit einer anderen der zwei Rillen (452) zu paaren und entlang dieser zu gleiten, wobei die zwei Zähne (446) voneinander um einen Umfang des proximalen Endes (426) des Freigabeknopfes (136, 404) beabstandet sind, und wobei das Gewinde der Antriebsspindel (161, 406) eine Steigung im Bereich von 2,54 bis 4,45 cm (1 bis 1,75 Zoll) hat.

## Revendications

1. Appareil de pose (100) pour un dispositif médical implantable déployable (10), l'appareil de pose comprenant :
une partie poignée (132, 400) comprenant un mécanisme de libération (200, 402) conçu pour ajuster une position linéaire, par rapport à un axe longitudinal central (L-L') de l'appareil de pose (100), d'un composant de l'appareil de pose (100), le mécanisme de libération (200, 402) comprenant :
une vis d'entraînement filetée (161, 406) comprenant une partie filetée hélicoïdale présentant un pas (466) d'au moins 2,54 cm (un pouce), la partie filetée hélicoïdale comprenant une ou plusieurs rainures (452) s'étendant autour de la vis d'entraînement (161, 406), la vis d'entraînement (161, 406) étant accouplée au composant ; et
un bouton rotatif (136, 404) entourant et coaxial avec la vis d'entraînement (161, 406), le bouton (136, 404) comprenant une ou plusieurs dents (446) agencées au niveau d'une extrémité proximale du bouton (136, 404), chaque dent (446) parmi ladite une ou lesdites plusieurs dents (446) étant conçue pour s'interfacer avec une rainure correspondante parmi ladite une ou lesdites plusieurs rainures (452) de la vis d'entraînement (161, 406), chaque dent (446) parmi ladite une ou lesdites plusieurs dents (446) s'étendant à partir de l'extrémité proximale, vers une extrémité distale (424) du bouton (136, 404), uniquement sur une partie d'une distance totale entre l'extrémité proximale (426) et l'extrémité distale (424), la partie étant inférieure à 1/4 de la distance totale (448).

2. Appareil de pose (100) selon la revendication 1, la vis d'entraînement filetée (161, 406) comprenant un ou plusieurs éléments de retenue (458) agencés au niveau d'une extrémité proximale (476) de la vis d'entraînement (161, 406), ladite une ou lesdites plusieurs rainures (452) de la partie filetée hélicoïdale étant chacune reliée à un élément de retenue (458) correspondant parmi ledit un ou lesdits plusieurs éléments de retenue (458) et s'étendant autour de la vis d'entraînement (161, 406), à partir de l'élément de retenue (458) correspondant vers une extrémité distale (474) de la vis d'entraînement (161, 406) et chaque dent (446) parmi ladite une ou lesdites plusieurs dents (446) étant conçue pour s'interfacer avec un élément de retenue (458) correspondant.

3. Appareil de pose (100) selon la revendication 2, chaque dent (446) étant conçue pour s'accoupler avec et se déplacer le long de la rainure (452) correspondante, lorsque le bouton (136, 404) est tourné autour de l'axe longitudinal central (L-L'), la vis d'entraînement (161, 406) étant conçue pour se déplacer linéairement, dans une direction axiale par rapport au bouton (136, 404), lorsque le bouton (136, 404) tourne et la dent (446) se déplace le long de la rainure (452) correspondante, le bouton (136, 404) étant fixé en translation dans la direction axiale, la direction axiale étant par rapport à l'axe longitudinal central (L-L'), le bouton (136, 404) comprenant un collier (422) s'étendant de manière distale à partir de l'extrémité distale (424) du bouton (136, 404) dans un intérieur d'un boîtier (410) de la partie poignée (132, 400), le collier (422) comprenant une ou plusieurs rainures (434) de collier s'étendant autour d'une circonférence du collier (422), chaque rainure (434) de collier s'accouplant avec une saillie annulaire (436) correspondante s'étendant radialement à partir d'une surface interne du boîtier (410) de la partie poignée (132, 400) et le bouton (136, 404) étant fixé en translation dans la direction axiale et étant conçu pour tourner autour de l'axe longitudinal central (L-L'), par rapport au boîtier (410) de la partie poignée (132, 400), par l'intermédiaire d'une liaison d'accouplement entre chaque rainure (434) de collier et la saillie annulaire (436) correspondante.

4. Appareil de pose (100) selon la revendication 2 ou la revendication 3, la vis d'entraînement (161, 406) étant mobile linéairement entre une configuration verrouillée de départ, où chaque dent (446) est accouplée avec l'élément de retenue (458) correspondant et une totalité de la partie filetée hélicoïdale de la vis d'entraînement (161, 406) est agencée à l'intérieur du bouton (136, 404) et de la partie poignée (132, 400), et une configuration libérée, où chaque dent (446) est accouplée avec une partie distale de la rainure (452) correspondante, la partie distale étant agencée plus près de l'extrémité distale (474) que l'extrémité proximale (476) de la vis d'entraînement (161, 406) et une majorité de la partie filetée hélicoïdale de la vis d'entraînement (161, 406) s'étendant vers l'extérieur de l'extrémité proximale (426) du bouton (136, 404), dans la direction axiale.

5. Appareil de pose (100) selon la revendication 4, chaque élément de retenue (458) comprenant un élément en saillie (460), une première partie de filetage linéaire (462) agencée sur un premier côté de l'élément en saillie (460) et une seconde partie de filetage linéaire (464) agencée sur un second côté de l'élément en saillie (460), la seconde partie de filetage linéaire (464) étant reliée à et continue avec une rainure (452) correspondante parmi ladite une ou lesdites plusieurs rainures (452) de la vis d'entraînement (161, 406) et, dans la configuration verrouillée de départ, chaque dent étant agencée à l'intérieur de la première partie de filetage linéaire (462) de l'élément de retenue (458) correspondant.

6. Appareil de pose (100) selon l'une quelconque des revendications 2 à 5, comprenant en outre une tige (499) comprenant une extrémité distale accouplée de manière fixe avec une surface interne de la partie poignée (132, 400) et la vis d'entraînement (161, 406) comprenant une partie d'extension (490) s'étendant axialement vers l'extérieur à partir de l'extrémité distale (474) de la vis d'entraînement (161, 406), la partie d'extension (490) comprenant un trou interne (494) monté autour de la tige (499) et conçu pour coulisser linéairement le long de la tige (499),
éventuellement une extrémité proximale de la tige (499) comprenant un élément d'arrêt (497) qui est plus large qu'une plus grande largeur du trou interne de la vis d'entraînement (161, 406) et l'élément d'arrêt (497) étant agencé à l'intérieur d'une cavité ouverte (495) d'un intérieur de la vis d'entraînement (161, 406), la cavité ouverte (495) étant agencée entre l'extrémité proximale (476) et l'extrémité distale (474) de la vis d'entraînement (161, 406).

7. Appareil de pose (100) selon la revendication 6, la partie d'extension (490) comprenant en outre un trou central (492) centré le long de l'axe longitudinal central (L-L'), le trou interne étant radialement décalé par rapport au trou central (492) et le trou central (492) étant conçu pour recevoir un arbre interne (152) de l'appareil de pose (100) à travers celui-ci.

8. Appareil de pose (100) selon l'une quelconque des revendications 1 à 7, le composant de l'appareil de pose (100), pour l'ajustement de la position linéaire duquel le mécanisme de libération (200, 402) est conçu, comprenant un ou plusieurs éléments de libération (156) accouplés de manière amovible au dispositif médical implantable (10),
éventuellement comprenant en outre un arbre interne (152, 488) comprenant une extrémité proximale accouplée de manière fixe avec un capuchon (408) du mécanisme de libération (200, 402), le capuchon (408) étant accouplé avec l'extrémité proximale (476) de la vis d'entraînement (161, 406), l'arbre interne s'étendant jusqu'à une extrémité distale de l'appareil de pose (100) et ledit un ou lesdites plusieurs éléments de libération (156) étant accouplés de manière fixe avec une partie d'extrémité distale de l'arbre interne (152, 488).

9. Appareil de pose (100) selon l'une quelconque des revendications 1 à 8, des filetages formés par ladite une ou lesdites plusieurs rainures (452) de la partie filetée hélicoïdale étant des filetages à double début formés par deux rainures (452) et la vis d'entraînement (161, 406) présentant deux éléments de retenue (458), chaque rainure (452) s'étendant à partir d'un élément différent parmi lesdits deux éléments de retenue (458) et le bouton (136, 404) comprenant deux dents (446), chacune étant conçue pour s'accoupler avec et coulisser le long d'une rainure différente parmi lesdites deux rainures (452), lesdites deux dents (446) étant écartées l'une de l'autre autour d'une circonférence de l'extrémité proximale (426) du bouton (136, 404).

10. Appareil de pose (100) selon l'une quelconque des revendications 1 à 9, la partie poignée (132, 400) comprenant en outre un mécanisme de direction comprenant un bouton de direction (418) conçu pour tourner par rapport à un boîtier (410) de la partie poignée (132, 400) et pour ajuster une courbure d'un ou de plusieurs arbres (134, 150, 152) de l'appareil de pose (100), au niveau d'une partie d'extrémité distale de l'appareil de pose (100).

11. Appareil de pose (100) selon l'une quelconque des revendications 1 à 10, le dispositif médical implantable (10) étant une valvule cardiaque prothétique conçue pour s'autodéployer radialement à une dimension fonctionnelle de la valvule cardiaque prothétique.

12. Appareil de pose (100) selon l'une quelconque des revendications 1 à 11, la partie de la distance totale (448) entre l'extrémité proximale (426) et l'extrémité distale (424) étant inférieure à 1/10 de la distance totale (448).

13. Appareil de pose (100) selon la revendication 1, l'appareil de pose (100) comprenant en outre :
un arbre interne (152, 488) ; et
un ou plusieurs éléments de libération (156, 208), chacun comprenant une extrémité proximale accouplée avec une surface externe d'une partie d'extrémité distale de l'arbre interne (152, 488) et une extrémité distale conçue pour être accouplée de manière libérable au dispositif médical implantable (10) agencé autour de la partie d'extrémité distale de l'arbre interne (152, 488), de manière distale par rapport à l'endroit où l'extrémité proximale s'accouple avec l'arbre interne (152, 488) ;
la partie poignée (132, 400) comprenant en outre :
un mécanisme de direction conçu pour ajuster une courbure d'un ou de plusieurs arbres (134, 150, 152) de l'appareil de pose (100) et le(s) fléchir, comprenant l'arbre interne (152, 488), au niveau d'une partie d'extrémité distale de l'appareil de pose (100) ;
le mécanisme de libération (200, 402) étant conçu pour ajuster une position linéaire de l'arbre interne (152, 488) et dudit un ou desdits plusieurs éléments de libération (156), le long d'un axe longitudinal central (L-L') de l'appareil de pose (100), par rapport à un boîtier externe (410) de la partie poignée (132, 400) ;
la vis d'entraînement filetée (161, 406) étant accouplée avec une extrémité proximale de l'arbre interne (152, 488) et la partie filetée hélicoïdale étant agencée dans un corps principal (456) de la vis d'entraînement (161, 406), ladite une ou lesdites plusieurs rainures (452) formant la partie filetée hélicoïdale s'étendant à partir d'une extrémité proximale (476) vers une extrémité distale (474) du corps principal (456) de la vis d'entraînement (161, 406) ; et
le bouton rotatif (136, 404) comprenant un bouton de libération rotatif (136, 404) accouplé au boîtier externe (410) de la partie poignée (132, 400) et la vis d'entraînement (161, 406) étant conçue pour se déplacer linéairement, le long de l'axe longitudinal central (L-L'), par rapport au bouton de libération (136, 404), en réponse à la rotation du bouton de libération (136, 404) et au coulissement de ladite une ou desdites plusieurs dents (446) le long de ladite une ou desdites plusieurs rainures (452) et l'actionnement du mécanisme de direction pour déplier ledit un ou lesdits plusieurs arbres (134, 150, 152) de l'appareil de pose (100) permettant à la vis d'entraînement (161, 406) de se déplacer de manière distale, le long de l'axe longitudinal central (L-L'), afin de libérer la tension créée dans la partie d'extrémité distale de l'appareil de pose (100),
éventuellement chaque dent (446) parmi ladite une ou lesdites plusieurs dents (446) du bouton de libération (136, 404) étant inférieure à un filetage complet et s'incurvant de moins de 45 degrés autour d'une circonférence de l'extrémité proximale (426) du bouton de libération (136, 404).

14. Appareil de pose (100) selon la revendication 13, le corps principal (456) de la vis d'entraînement (161, 406) comprenant en outre un ou plusieurs éléments de retenue (458) agencés au niveau de l'extrémité proximale (476) du corps principal (456), chaque rainure (452) parmi ladite une ou lesdites plusieurs rainures (452) de la partie filetée hélicoïdale étant reliée à un élément de retenue (458) correspondant dudit un ou desdits plusieurs éléments de retenue (458) et s'incurvant autour d'une surface externe du corps principal (456) et s'étendant à partir de l'élément de retenue (458) vers l'extrémité distale (474) du corps principal (456),
éventuellement la vis d'entraînement (161, 406) étant mobile linéairement entre une configuration verrouillée de départ, où chaque dent (446) parmi ladite une ou lesdites plusieurs dents (446) est accouplée avec un élément de retenue (458) correspondant parmi ledit un ou lesdits plusieurs éléments de retenue (458) et une totalité de la partie filetée hélicoïdale de la vis d'entraînement (161, 406) est agencée à l'intérieur du bouton de libération (136, 404) et de la partie poignée (132, 400), et une configuration libérée, où chaque dent (446) est accouplée avec une partie distale d'une rainure (452) correspondante parmi ladite une ou lesdites plusieurs rainures (452), la partie distale étant agencée plus près de l'extrémité distale que l'extrémité proximale (426) du corps principal (428) et une majorité de la partie filetée hélicoïdale de la vis d'entraînement (161, 406) s'étendant vers l'extérieur de l'extrémité proximale (426) du bouton de libération (136, 404), dans une direction axiale.

15. Appareil de pose (100) selon la revendication 13 ou la revendication 14, les filetages formés par ladite une ou lesdites plusieurs rainures (452) de la partie filetée hélicoïdale étant des filetages à double début formés par deux rainures (452), le bouton de libération (136, 404) comprenant deux dents (446), chacune étant conçue pour s'accoupler avec et coulisser le long d'une rainure différente parmi lesdites deux rainures (452), lesdites deux dents (446) étant écartées l'une de l'autre autour d'une circonférence de l'extrémité proximale (426) du bouton de libération (136, 404) et les filetages de la vis d'entraînement (161, 406) présentant un pas dans une plage de 2,54 à 4,45 cm (1 à 1,75 pouces).
